(19) Europäisches Patentamt
European Patent Office
Office européen des brevets

(11) Publication number: **0 085 291**
**B1**

# (12) EUROPEAN PATENT SPECIFICATION

(45) Date of publication of patent specification: **17.08.88**

(21) Application number: **83100006.2**

(22) Date of filing: **03.01.83**

(51) Int. Cl.[4]: **C 07 D 205/08,**
C 07 D 403/12,
C 07 D 403/14,
C 07 D 413/14,
C 07 D 417/12,
C 07 D 417/14, A 61 K 31/64

## (54) 2-Oxo-1-(aminocarbonylaminosulfonylaminocarbonyl)azetidines.

(30) Priority: **04.01.82 US 336537**
**15.04.82 US 368609**

(43) Date of publication of application:
**10.08.83 Bulletin 83/32**

(45) Publication of the grant of the patent:
**17.08.88 Bulletin 88/33**

(84) Designated Contracting States:
**AT BE CH DE FR GB IT LI LU NL SE**

(56) References cited:
**EP-A-0 062 876**

**Chemical Abstracts vol. 83, no. 11, 1975, Columbus, Ohio (US); DH.AUE et al.: "Addition of p-toluenesulfonyl to imino ethers. Isolation of a stable1,4-dipolar intermediate", p. 468, l/h column, abstract 95909d**

(73) Proprietor: **E.R. Squibb & Sons, Inc.**
**Lawrenceville-Princeton Road**
**Princeton, N.J. 08540 (US)**

(72) Inventor: **Breuer, Hermann**
**Sauerzapfstrasse 5**
**D-8411 Schoenhofen (DE)**
Inventor: **Denzel, Theodor**
**Lessingstrasse 12**
**D-8400 Regensburg (DE)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 085 291 B1

**Description**

β-Lactams having the formula

I

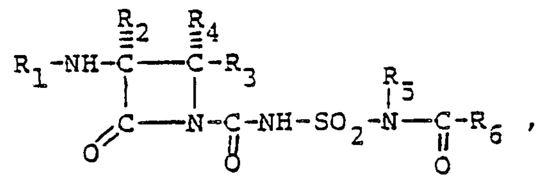

and salts thereof, have antibacterial activity. In formula I, and throughout the specification, the symbols are as defined below.

$R_1$ is an acyl group commonly employed in betalactam antibiotics;

$R_2$ is hydrogen or methoxy;

$R_3$ and $R_4$ are the same or different and each is hydrogen, alkyl, or aryl;

$R_5$ is hydrogen, alkyl or aryl;

$R_6$ is hydrogen, alkyl, aryl, a 5, 6 or 7-membered heterocycle, $—NR_7R_8$, or $—(CH_2)_n—X$ wherein n is 1, 2, 3 or 4 and X is halogen, aryl, alkoxy, aryloxy or $—NR_9R_{10}$;

$R_7$ and $R_8$ are the same or different and each is hydrogen, alkyl or aryl, or $R_7$ is hydrogen and $R_8$ is a 5,6 or 7-membered heterocycle or $—(CH_2)_n—Y$ wherein n is 1,2,3 or 4 and Y is alkoxy, amino($—NH_2$), alkylthio or halogen; and

$R_9$ and $R_{10}$ are the same or different and each is hydrogen or alkyl, or $R_9$ is hydrogen and $R_{10}$ is a 5,6 or 7-membered heterocycle.

Listed below are definitions of various terms used to describe the β-lactams of this invention. These definitions apply to the terms as they are used throughout the spefification (unless they are otherwise defined in specific instances) either individually or as part of a larger group.

The terms "alkyl" and "alkoxy" refer to both straight and branched chain groups having 1 to 10 carbon atoms.

The term "halogen" refers to fluorine, chlorine, bromine and iodine.

The term "protected carboxyl" refers to a carboxyl group which has been esterified with a conventional acid protecting group. These groups are well known in the art; see, for example, United States patent 4,144,333, issued March 13, 1979. The preferred protected carboxyl groups are benzyl, benzhydryl, *t*-butyl, and *p*-nitrobenzyl esters.

The term "aryl" refers to phenyl and phenyl substituted with 1,2 or 3 amino($—NH_2$), halogen, hydroxyl, trifluoromethyl, alkyl (of 1 to 4 carbon atoms), alkoxy (of 1 to 4 carbon atoms) or carboxyl groups.

The expression "a 5, 6 or 7-membered heterocycle" refers to aromatic and non-aromatic groups containing one or more nitrogen, oxygen or sulfur atoms which can bear (provided $R_6$ is $NR_7R_8$ or a heterocycle) oxo(=O), halogen, hydroxy, nitro, amino, cyano, trifluoromethyl, alkyl of 1 to 4 carbons, alkoxy of 1 to 4 carbons, alkylsulfonyl, phenyl, phenyl substituted with 1, 2 or 3 amino ($—NH_2$), halogen, hydroxyl, triluoromethyl, $C_1—C_4$alkyl, $C_1—C_4$ alkoxy or carboxyl groups, 2-furylmethyleneimino

(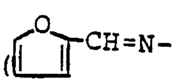 ),

phenylmethyleneimino and substituted $C_1—C_4$ alkyl substituents.

One type of "5,6 or 7-membered heterocycle" is the "heteroaryl" group. The term "heteroaryl" refers to those 5,6 or 7-membered heterocycles which are aromatic. Exemplary heteroaryl groups are substituted and unsubstituted pyridinyl, furanyl, pyrrolyl, thienyl, 1,2,3-triazolyl, 1,2,4-triazolyl, imidazolyl, thiazolyl, thiadiazolyl, pyrimidinyl, oxazolyl, triazinyl, and tetrazolyl. Exemplary nonaromatic heterocycles (*i.e.*, fully or partially saturated heterocyclic groups) are substituted and unsubstituted piperidinyl, piperazinyl, imidazolidinyl, oxazolidinyl, pyrrolidinyl, tetrahydropyrimidinyl, and dihydrothiazolyl. Exemplary of the substituted 5,6 or 7-membered heterocycles are 2-oxo-1-imidazolidinyl, 3-alkylsulfonyl-2-oxo-1-imidazolidinyl, 3-benzylimino-2-oxo-1-imidazolidinyl, 3-alkyl-2-oxo-1-imidazolidinyl, 3-aryl-2-oxo-1-imidazolidinyl, 3-(2-hydroxyethyl)-2-oxo-1-imidazolidinyl, 3-(2-aminoethyl)-2-oxo-1-imidazolidinyl, 3-amino-2-oxo-1-imidazolidinyl, 2-oxo-1-pyrrolidinyl, 2-oxo-3-oxazolidinyl, 4-hydroxy-6-methyl-2-pyrimidinyl, 2-oxo-3-pyrrolidinyl, 2-oxo-3-tetrahydrofuranyl, 2,3-dioxo-1-piperazinyl, 2,5-dioxo-1-piperazinyl, 4-alkyl-2,3-dioxo-1-piperazinyl, and 4-phenyl-2,3-dioxo-1-piperazinyl.

The term "substituted alkyl" refers to alkyl groups substituted with one, or more, azido, amino($—NH_2$), halogen, hydroxy, carboxy, cyano, alkoxycarbonyl, aminocarbonyl, alkanoyloxy, having 2 to 10 carbon atoms, alkoxy, aryloxy, a 5,6 or 7-membered heterocycleoxy which is unsubstituted, mercapto, alkylthio, arylthio, alkylsulfinyl, or alkylsulfonyl groups.

The term "acyl" refers to all organic radicals derived from an organic acid (*i.e.*, a carboxylic acid) by removal of the hydroxyl group, which have been used in the past to acylate β-lactam antibiotics including

6-aminopenicillanic acid and derivatives and 7-aminocephalosporanic acid and derivatives; see, for example, *Cephalosporins and Penicillins*, edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 3,971,778, issued July 27, 1976, United States patent 4,172,199, issued October 23, 1979, and British patent 1,348,894, published March 27, 1974. The portions of these references describing various acyl groups are incorporated herein by reference. The following list of acyl groups is presented to further exemplify the term "acyl"; is should not be regarded as limiting that term. Exemplary acyl groups are:

(a) Aliphatic groups having the formula

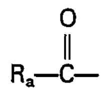

wherein $R_a$ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

(b) Carbocyclic aromatic groups having the formula

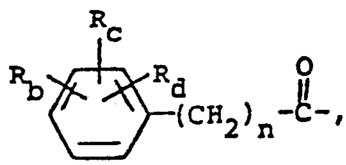

,

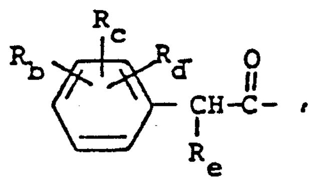

,

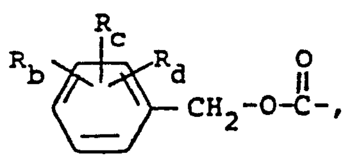

,

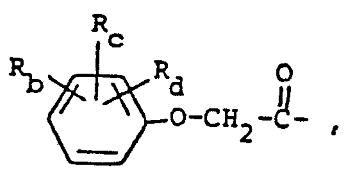

,

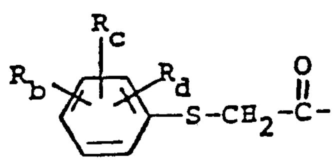

or

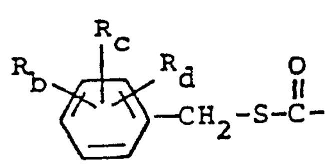

wherein n is 0, 1, 2 or 3; $R_b$, $R_c$, and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

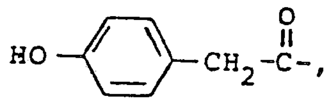

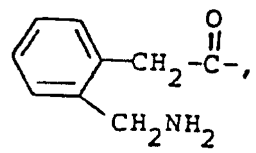

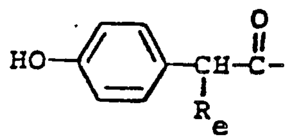

($R_e$ is preferably a carboxyl salt or sulfo salt) and

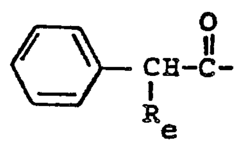

($R_e$ is preferably a carboxyl salt or sulfo salt).

(c) Heteroaromatic groups having the formula

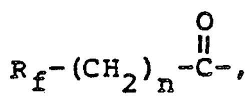

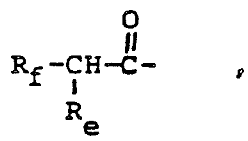

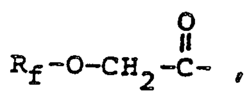

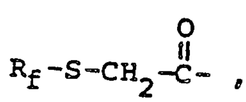

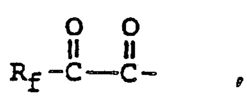

wherein n is 0, 1, 2 or 3; $R_e$ is as defined above; and $R_f$ is a substituted or unsubstituted 5-, 6- or 7-membered heterocyclic ring containing 1,2,3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, thiadiazolyl, pyrimidinyl and tetrazolyl. Exemplary substituents are halogen, hydroxyl, nitro, amino, cyano,

trifluoromethyl, alkyl of 1 to 4 carbon atoms, alkoxy of 1 to 4 carbon atoms, or

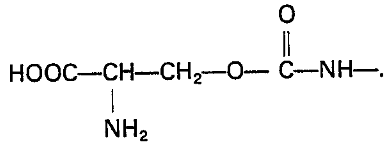

Preferred heteroaromatic acyl groups include those groups of the above formulas wherein $R_f$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

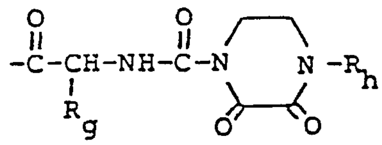

wherein $R_g$ is an aromatic group (including carbocyclic aromatics such as those of the formula

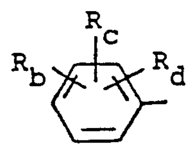

and heteroaromatics as included within the definition of $R_f$); and $R_h$ is alkyl, substituted alkyl (wherein the alkyl group is substituted with one or more halogen, cyano, nitro, amino or mercapto groups), arylmethyleneamino (*i.e.,* $-N{=}CH-R_g$ wherein $R_g$ is as defined above), arylcarbonylamino (*i.e,*

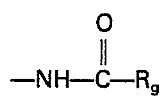

wherein $R_g$ is as defined above) or alkylcarbonylamino.

Preferred [[(4-substituted-2,3-dioxo-1-piperazinyl)carbonyl]amino]arylacetyl groups include those wherein $R_h$ is ethyl, phenylmethyleneamino or 2-furylmethyleneamino.

(e) (Substituted oxyimino)arylacetyl groups having the formula

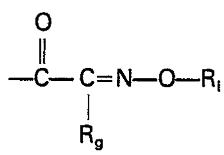

wherein $R_g$ is as defined above and $R_i$ is hydrogen, alkyl, cycloalkyl, alkylaminocarbonyl, arylaminocarbonyl (*i.e.,*

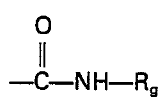

wherein $R_g$ is as defined above) or substituted alkyl (wherein the alkyl group is substituted with 1 or more halogen, cyano, nitro, amino, mercapto, alkylthio, aromatic group (as defined by $R_g$), carboxyl (including salts thereof), amido, alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl, or dialkoxyphosphinyl substituents).

Preferred (substituted oxyimino)arylacetyl groups include those wherein $R_g$ is 2-amino-4-thiazolyl. Also preferred are those groups wherein $R_i$ is methyl, ethyl, carboxymethyl, 1-carboxy-1-methylethyl or 2,2,2-trifluoroethyl.

(f) (Acylamino)arylacetyl groups having the formula

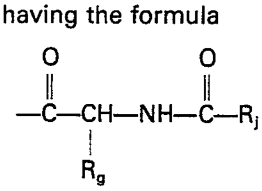

wherein $R_g$ is as defined above and $R_j$ is

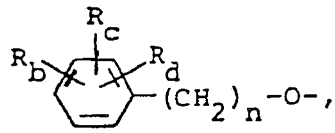

amino, alkylamino, (cyanoalkyl)amino, amido, alkylamido, (cyanoalkyl)amido,

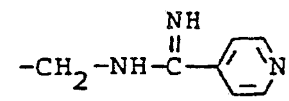

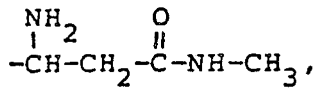

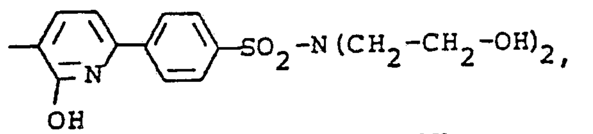

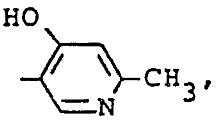

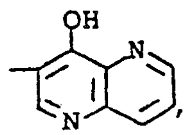

or

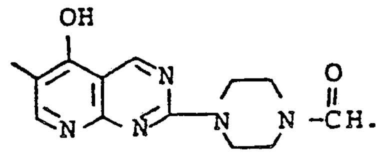

Preferred (acylamino)arylacetyl groups of the above formula include those groups wherein $R_j$ is amino or amido. Also preferred are those groups wherein $R_g$ is phenyl or 2-thienyl.

(g) [[[3-Substituted-2-oxo-1-imidazolidinyl]carbonyl]amino]arylacetyl groups having the formula

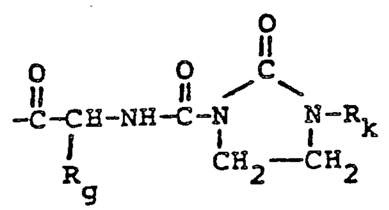

wherein $R_g$ is as defined above and $R_k$ is hydrogen, alkylsulfonyl, arylmethyleneamino (*i.e.*, —N=CH—$R_g$ wherein $R_g$ is as defined above),

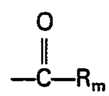

(wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups).

Preferred [[[3-Substituted-2-oxo-1-imidazolidinyl]carbonyl]amino]arylacetyl groups of the above formula include those wherein $R_g$ is phenyl or 2-thienyl. Also preferred are those groups wherein $R_k$ is hydrogen, methylsulfonyl, phenylmethyleneamino or 2-furylmethyleneamino.

The compounds of this invention form basic salts with various inorganic and organic bases which are also within the scope of this invention. Such salts include ammonium salts, alkali metal salts, alkaline earth metal salts, salts with organic bases, *e.g.*, dicyclohexylamine, benzathine, N-methyl-D-glucamine, hydrabamine and the like. The pharmaceutically acceptable salts are preferred, although other salts are also useful, *e.g.*, in isolating or purifying the product.

Some of the compounds of this invention may be crystallized or recrystallized from solvents containing water. In these cases water of hydration may be formed. This invention contemplates stoichiometric hydrates as well as compounds containing variable amounts of water that may be produced by processes such as lyophilization.

β-Lactams having a

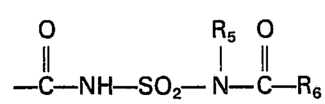

substituent in the 1-position and an acylamino substituent in the 3-position contain at least one chiral center — the carbon atom (in the 3-positon of the β-lactam nucleus) to which the acylamino substituent is attached. This invention is directed to those β-lactams which have been described above, wherein the stereochemistry at the chiral center in the 3-position of the β-lactam nucleus is the same as the configuration at the carbon atom in the 6-position of naturally occurring pencillins (*e.g.*, penicillin G) and as the configuration at the carbon atom in the 7-position of naturally occurring cephamycins (*e.g.*, cephamycin C).

Also included within the scope of this invention are racemic mixtures which contain the above-described β-lactams.

β-Lactams having a

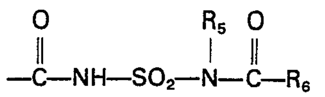

substituent in the 1-position of the β-lactam nucleus and an acylamino substituent in the 3-position of the β-lactam nucleus, and salts thereof, have activity against a range of gram-negative and gram-positive organisms.

The compounds of this invention can be used as agents to combat bacterial infections (including urinary tract infections and respiratory infections) in mammalian species, such as domesticated animals (*e.g.*, dogs, cats, cows, horses, and the like) and humans.

For combating bacterial infections in mammals a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with the novel family of β-lactams of this invention. Such methods of administration include, oral, intravenous, intramuscular, and as a suppository.

A

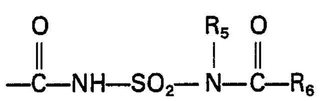

activating group can be introduced onto the nitrogen atom of a β-lactam by reacting a β-lactam having the formula

II

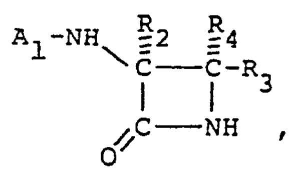

wherin $A_1$ is a nitrogen protecting group, with the appropriate isocyanate having the formula

III

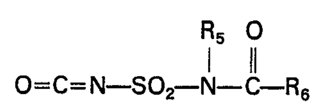

As the protective group $A_1$ for amino above, any of those used for this purpose in the field of β-lactam or peptide synthesis may conveniently be employed. Examples of such amino protecting group include aromatic acyl groups such as phthaloyl, p-nitrobenzoyl, p-tert-butylbenzoyl, p-tertbutylbenzenesulfonyl, benzenesulfonyl, toluenesulfonyl, etc., aliphatic acyl groups such as formyl, acetyl, propionyl, monochloroacetyl, dichloroacetyl, trichloroacetyl, methanesulfonyl, ethanesulfonyl, trifluoroacetyl, maleyl, succinyl, etc., and esterifed carboxyl groups such as methoxycarbonyl, ethoxycarbonyl, t-butoxycarbonyl, isopropoxycarbonyl, 2-cyanoethoxycarbonyl, β,β,β-trichloroethoxycarbonyl, β-trimethylsilylethoxycarbonyl, β-methylsulfonylethoxycarbonyl, benzyloxycarbonyl, p-nitrobenzyloxycarbonyl, p-methoxybenzyloxycarbonyl, di-phenylmethyloxycarbonyl, methoxymethyloxycarbonyl, acetylmethyloxycarbonyl, isobornyloxycarbonyl, phenyloxycarbonyl, etc., as well as nonacyl amino-protecting groups such as trityl, 2-nitrophenylthio, benzylidene, 4-nitrobenzylidene, trialkylsilyl, benzyl, p-nitrobenzyl, proton, etc. The choice of amino-protecting group is not critical in the present invention.

The reaction is preferably run in an organic solvent, e.g., an inert solvent such as tetrahydrofuran or dimethoxyethane, in the presence of a base such as triethylamine or alkyl lithium.

The activating group can also be inserted onto the Formula II β-lactam in sequential segments. Thus for example, with reference to the activating group as represented below:

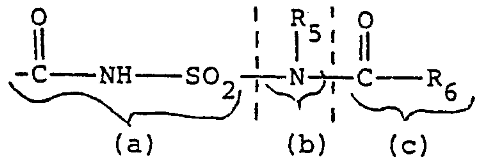

there are various methods known in the art by which the sequential portions (a), (b) and (c) above can be added to the Formula II β-lactam in single or double bond portions or all together.

One such method, which is preferred comprises first reacting a β-lactam of formula II with an isocyanate having the formula

IV $$O{=}C{=}N{-}SO_2{-}Z$$

wherein Z is a leaving group, *e.g.*, a halogen such as chlorine. The reaction is preferably run in an inert organic solvent, *e.g.*, a halocarbon such as dichloromethane, or in acetonitrile, and yields an intermediate having the formula

V

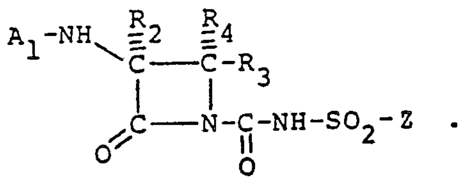

Reaction of an intermediate of formula V with a $R_5NH_2$ amine preferably in the form of a silylated derivative such as with a silyl compound having the formula

VI

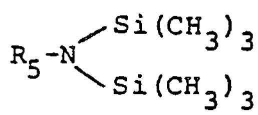

yields an intermediate having the formula

VII

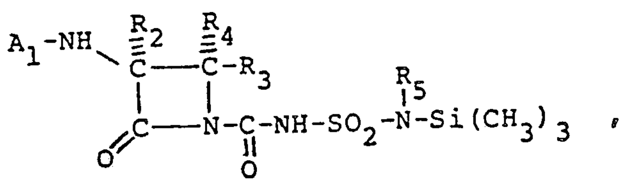

which can be reacted with an acyl halide having the formula

VIII

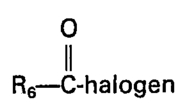

to yield a β-lactam having the formula

IX

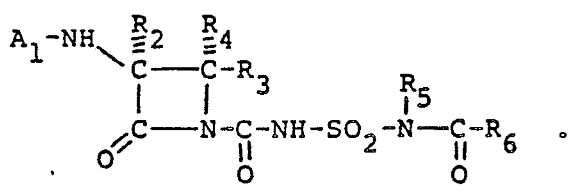

Alternatively, compounds of formulas VI and VIII can be first reacted to yield a compound having the formula

X

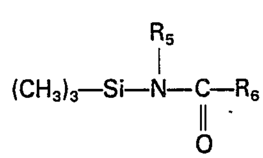

which can be reacted with a compound of formula V to yield a compound of formula IX.

Still another procedure for preparing a compound of formula IX wherein $R_6$ is —$NR_7R_8$, comprises reacting a compound of formula V with a urea having the formula

XI

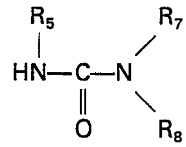

in the presence of triethylamine.

Deprotection of a compound of formula IX using conventional techniques yields the corresponding key intermediate having the formula

XII

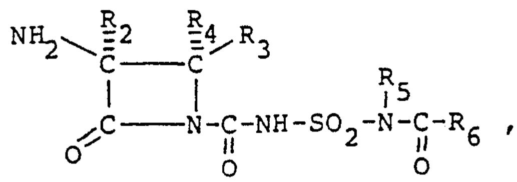

or a salt thereof. The particular deprotection reaction used will, of course, depend on the protecting group ("$A_1$") present. If, for example, $A_1$ is a *t*-butoxycarbonyl protecting group, deprotection can be accomplished by treatment of a compound of formula IX with acid (*e.g.*, formic acid or trifluoroacetic acid). If, for example, $A_1$ is a benzyloxycarbonyl protecting group, deprotection can be accomplished by catalytic hydrogenation of a compound of formula IX.

Well known acylation techniques can be used to convert an intermediate of formula XII to a corresponding product of formula I. It is also evident that the desired acyl group in the Formula I product can also function as the protecting group for the amino group ($NH_2$—) in the Formula XII compound above, or as the $A_1$-group in the Formula II reactant shown previously where $A_1$-is defined as a nitrogen protecting group. In such cases, there will be no need for removing the $A_1$-protecting group after insertion of the

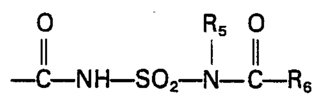

activating group.

Exemplary techniques include reaction of a compound of formula XII with a carboxylic acid ($R_1$—OH), or corresponding carboxylic acid halide or carboxylic acid anhydride. The reaction with a carboxylic acid proceeds most readily in the presence of a carbodiimide such as dicyclohexylcarbodiimide and a substance capable of forming an active ester *in situ* such as N-hydroxybenzotriazole. In those instances where the acyl group ($R_1$) contains reactive functionality (such as amino or carboxyl groups) it may be necessary to first protect those functional groups, then carry out the acylation reaction, and finally deprotect the resulting product.

In general terms, the process for preparing the desired compounds therefore comprises reacting a β-lactam having the formula

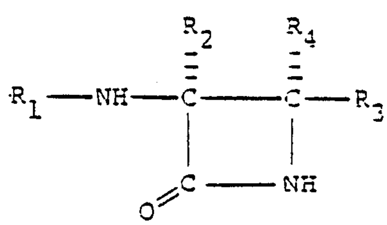

wherein $R_2$, $R_3$ and $R_4$ are as previously defined and $R_1'$ is acyl ($R_1$) as previously defined or the group $R_1'$—NH— is a protected amino group, with an isocyanate having the formula

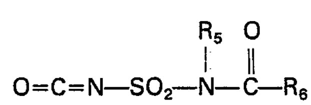

or sequential segments thereof wherein $R_5$ and $R_6$ are as previously defined, and where $R_1'$—NH— is a protected amino group removing said protecting group and acylating with the appropriate acylating group to form the desired product.

The azetidinones of formula II can be prepared utilizing the procedures described in United Kingdom patent application 2,071,650, published September 23, 1981.

The preferred compounds of formula I are those wherein $R_3$ and $R_4$ are each hydrogen. Also preferred are those compounds of formula I wherein $R_6$ is a 5,6 or 7-membered heterocycle, especially a 4-alkyl-2,3-dioxo-1-piperazinyl group, a 2-oxo-1-imidazolidinyl group, a 3-alkyl-2-oxo-1-imidazolidinyl group or a 3-(substituted alkyl)-2-oxo-1-imidazolidinyl group. Specific groups that are preferred are the 4-ethyl-2,3-dioxo-1-piperazinyl, 3-ethyl-2-oxo-1-imidazolidinyl, and 3-(2-aminoethyl)-2-oxo-1-imidazolidinyl groups. Preferred 3-acylamino groups are those wherein the acyl portion of the group is (Z)-2-amino-α-(alkoxyimino)-4-thiazoleacetyl or (Z)-2-amino-α-[[(substituted alkyl)oxy]imino]-4-thiazoleacetyl, especially (Z)-2-amino-α-(methoxyimino)-4-thiazoleacetyl and (Z)-2-amino-α-[(1-carboxy-1-methylethoxy)imino]-4-thiazoleacetyl.

The following examples are specific embodiments of this invention.

## Example 1

### [3S(Z)]-N-[1-[[[(Acetylamino)sulfonyl]amino]-carbonyl]-2-oxo-3-azetidinyl]-2-amino-α-(methoxyimino)-4-thiazoleacetamide, dipotassium salt

A) (S)-[1-[[[(Acetylamino)sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (4.4 g) was suspended in dry ethyl acetate. The mixture was cooled to −5°C and 3.1 g of chlorosulfonyl isocyanate was dropped in with stirring at such a rate that the temperature did not exceed 0°C. Stirring at 0°C was continued for an additional 20 minutes. After this time 4 g of hexamethyldisilazane was added and the solution was stirred at ambient temperature for 12 hours. Acetyl chloride (3.2 g) was added and the solution was again stirred for 48 hours. The ethyl acetate was then washed with water and extracted twice with 50 ml portions of saturated aqueous bicarbonate. The organic layer was discarded and the aquoeus phase was treated with 25% hydrochloric acid until the pH was 1.0. Extraction with ethyl acetate, drying and evaporation of the solvent yielded 4 g of the title compound in crude form. This was dissolved in acetone/water (1:1), the pH was adjusted to 6.5, the solvent was removed *in vacuo* and the crystalline title compound was filtered off with ether. Further purification achieved by HP—20 chromatogrphy (water/acetone 9:1 as eluent) yielded the title compound, melting point 130—135°C, *dec.*

B) [3S(Z)]-N-[1-[[[(Acetylamino)sulfonyl]amino]carbonyl]-2-oxo-3-azetidinyl]-2-amino-α-(methoxyimino)-4-thiazoleacetamide, dipotassium salt

(S)-[1-[[[(Acetylamino)sulfonyl]amino]carbonyl]-2-oxo-3-azetidinyl]carbamic acid, phenylmethyl ester, potassium salt (940 mg) was dissolved in 50 ml of dry dimethylformamide and hydrogenated in the presence of 500 mg of 10% palladium on charcoal for 30 minutes, after which the catalyst was filtered off. (Z)-2-Amino-α-(methoxyimino)-4-thiazoleacetic acid (450 mg), 1 g of dicyclohexylcarbodiimide and 150 mg of N-hydroxybenzotriazole were added to the filtrate. The solution was stirred overnight, the precipitated urea was filtered off and the solvent was removed *in vacuo*. The remaining solid was chromatographed on HP—20* using water as eluent, and yielded 400 mg of product, melting point 255—260°C, *dec.*

* The terms "HP—20" and "HP—20 resin" refer to macroporous styrene-divinylbenzene copolymer.

## Example 2

### [3S(Z)]-2-[[[2-[[1-[[[(Acetylamino)sulfonyl]amino]carbonyl]-2-oxo-3-azetidinyl]amino]-1-(2-amino-4-thiazolyl)-2-oxoethylidene]amino]oxy]-2-methylpropanoic acid

A) 2-[[[2-[[1-[[[(Acetylamino)sulfonyl]amino]carbonyl]-2-oxo-3-azetidinyl]amino]-1-(2-amino-4-thiazolyl)-2-oxoethylidene]amino]oxy]-2-methylpropanoic acid, diphenylmethyl ester, monopotassium salt

(S)-[1-[[[(Acetylamino)sulfonyl]amino]carbonyl]-2-oxo-3-azetidinyl)carbamic acid, phenylmethyl ester, potassium salt (1.38 g; see example 1A) was hydrogenated in dry dimethylformamide in the presence of 700 mg of 10% palladium on charcoal for 30 minutes after which the catalyst was filtered off. (Z)-2-Amino-α-[[2-(diphenylmethoxy)-1,1-dimethyl-2-oxoethoxy]imino]-4-thiazoleacetic acid (1.32 g), 1.2 g of dicyclohexylcarbodiimide and 150 mg of N-hydroxybenzotriazole were added and the solution was stirred for 12 hours. The precipitated urea was filtered off and the solvent was removed *in vacuo*. The residue was dissolved in 20 ml of acetone, filtered and poured into 100 ml of ether. The precipitated title compound was filtered off and dried yielding 2.5 g of material.

B) [3S(Z)]-2-[[[2-[[1-[[[(Acetylamino)sulfonyl]amino]carbonyl]-2-oxo-3-azetidinyl]amino]-1-(2-amino-4-thiazolyl)-2-oxoethylidene]amino]oxy]-2-methylpropanoic acid

2-[[[2-[[1-[[[(Acetylamino)sulfonyl]amino]carbonyl]-2-oxo-3-azetidinyl]amino]-1-(2-amino-4-thiazolyl)-2-oxoethylidene]amino]oxy]-2-methylpropanoic acid, diphenylmethyl ester, monopotassium salt (2.5 g) was suspended in 5 ml of anisole and cooled to 0°C. Trifluoroacetic acid (12 ml) was slowly dropped in with stirring, maintaining the temperature at 0°C. After 3.5 hours, the solution was poured into 100 ml of ether, precipitating the desired product. The crude product was filtered off and purified by HP—20 chromatography using water/acetone (9:1) as eluent and yielding 620 mg of product, melting point 225—230°C, *dec.*

Example 3

[1-[[[[(Chloroacetyl)amino]sulfonyl]amino]carbonyl]-2-oxo-3-azetidinyl)carbamic acid, phenylmethyl ester

(S)-3-[[(Phenylmethoxy)carbonyl]amino]-2-azetidinone (4.4 g) was suspended in 150 ml of dry ethyl acetate. The mixture was cooled to −50°C and 3.3 g of chlorosulfonyl isocyanate was added with stirring; stirring was continued without cooling until the temperature reached 0°C. N-(Trimethylsilyl)chloroacetamide (6.0 g) was added and the solution was stirred overnight.

The insoluble material was filtered off and the filtrate washed with water. The organic layer was extracted twice with saturated aqueous sodium bicarbonate. The alkaline aqueous layer was acidified to pH 1 with 20% hydrochloric acid and extracted twice with 150 ml portions of ethyl acetate. The organic layers were combined, dried and evaporated to dryness. The oily residue was dissolved in 50 ml of acetone and the pH was adjusted to 6.5 by addition of 1N potassium hydroxide. The solvent was removed *in vacuo* and the crystalline residue filtered with ether, yielding 3.9 g of the title compound.

Example 4

[3S(R)-N-[2-[[1-[[[(Acetylamino)sulfonylamino]carbonyl]-2-oxo-3-azetidinyl]amino]-2-oxo-1-phenylethyl]-4-ethyl-2,3-dioxo-1-piperazinecarboxamide, potassium salt

Following the procedure of example 1B, but substituting (R)-α-[[(4-ethyl-2,3-dioxo-1-piperazinyl)-carbonyl]amino]benzeneacetic acid for (Z)-2-amino-α-(methoxyimino)-4-thiazoleacetic acid, yielded the title compound melting point 160—165°C, *dec.*

Example 5

[3S(Z)]-2-Amino-N-1-[[[[(chloroacetyl)amino]sulfonyl]amino]carbonyl]-2-oxo-3-azetidinyl]-α-(methoxyimino)-4-thiazoleacetamide, potassium salt

[1-[[[[(Chloroacetyl)amino]sulfonyl]amino]carbonyl]-2-oxo-3-azetidinyl]carbamic acid, phenylmethyl ester (1.0 g; see example 3) was slowly added to 20 ml of 40% HBr in acetic acid at 10°C. When the addition was complete the solution was stirred for 5 minutes. Dry diethyl ether (100 ml) was added and the precipitate was filtered off, washed with ether and dried carefully. This compound was dissolved in 50 ml of water, the pH adjusted to 6.5 with 1N KOH and the resulting solution freeze-dried. The compound obtained was dissolved in 50 ml of dry dimethylformamide and (Z)-2-amino-α-(methoxyimino)-4-thiazoleacetic acid, 0.04 g of N-hydroxybenzotriazole and 0.76 g dicyclohexylcarbodiimide are added and the solution was stirred overnight at ambient temperature. The precipitated urea was filtered off and the solvent removed *in vacuo*. The residue was chromatographed using HP—20 resin (eluent:water), yielding 0.19 g of product, melting point 215—220°C.

Example 6

[3S(Z)]-2-[[[1-(2-Amino-4-thiazolyl)-2-[[1-[[[[(chloroacetyl)amino]sulfonyl]amino]carbonyl]-2-oxo-3-azetidinyl]amino]-2-oxoethylidene]amino]oxy]-2-methylpropanoic acid, dipotassium salt

[1-[[[[(Chloroacetyl)amino]sulfonyl]amino]carbonyl]-2-oxo-3-azetidinyl]carbamic acid, phenylmethyl ester (2.0 g; see example 3) was added at 5°C with stirring to 40 ml of 40% hydrogen bromide in acetic acid; after 5 minutes a clear solution was obtained. Dry ether (200 ml) was slowly dropped in and the precipitate was filtered off, washed with ether and dried carefully. The white powder was then dissolved in 50 ml of water and the pH adjusted to 6.5 and the solution freeze-dried. The compound obtained was dissolved in 100 ml of dry dimethylformamide and 2.72 g of (Z)-2-amino-α-[[2-(diphenylmethoxy)-1.1-dimethyl-2-oxo-ethoxy]imino]-4-thiazoleacetic acid, 0.08 g N-hydroxybenzotriazole and 1.52 g of dicyclohexylcarbodiimide was added and the solution was stirred for 12 hours. The precipitate was filtered off and the solvent removed *in vacuo*. The residue was stirred with 100 ml of dry ether and filtered. The compound was then suspended in 6 ml of anisole and cooled to −15°C. At this point 12 ml of trifluoroacetic acid was dropped in with stirring at such a rate, that the temperature did not exceed −10°C. After completion of the addition, stirring was continued at −10°C for 2 hours. Cold ether (200 ml) was added and the precipitated compound was filtered off, washed with ether, dried and dissolved in 50 ml of acetone/water (1:1). The pH was adjusted to 6.5 with 1N KOH, the acetone removed *in vacuo* and the remaining aqueous solution freeze-dried; 2.25 g of crude title compound was obtained. Purification was achieved in HP—20 chromatography (water eluent), yielding 0.5 g of product, melting point 220—225°C, *dec.*

Example 7

[3S(Z)[-2-[[[1-(2-Amino-4-thiazolyl)-2-[[1-[[[[(methoxyacetyl)amino]sulfonyl]amino]carbonyl]-2-oxo-3-azetidinyl]amino]-2-oxoethylidene]amino]oxy]-2-methylpropoinic acid, dipotassium salt

A) [1-[[[[(Methoxyacetyl)amino]sulfonyl]amino]carbonyl]-2-oxo-3-azetidinyl]carbamic acid, phenylmethyl ester, monopotassium salt

(S)-3-[[(Phenylmethoxy)carbonyl]amino]-2-azetidinone (4.4 g) was suspended in 100 ml of dry ethyl acetate. The mixture was cooled to −30°C and 3.12 g of chlorosulfonylisocyanate was added with stirring. The cooling was stopped and when the temperature had reached 0°C a clear solution was obtained. After 30 minutes at 0°C 13.9 g of N-trimethylsilyl methoxyacetamide were added and stirring was continued for 24 hours at ambient temperature. After this time the ethyl acetate solution was washed with water, dried and the solvent removed. The residue was dissolved in acetone/water (9:1) and the pH was adjusted to 6.5 with 1N KOH. The acetone was removed *in vacuo* and the aqueous solution was freeze-dried.

B) [3S(Z)]-2-[[[1-(2-Amino-4-thiazolyl)-2-[[1-[[[[(methoxyacetyl)amino]sulfonyl]amino]carbonyl]-2-oxo-3-azetidinyl]amino]-2-oxo-ethylidene]amino]oxy]-2-methylpropanoic acid, dipotassium salt

(S)-[1-[[[[(Methoxyacetyl)amino]sulfonyl]amino]carbonyl]-2-oxo-3-azetidinyl]carbamic acid, phenylmethyl ester, monopotassium salt (0.43 g) was dissolved in 50 ml of dry dimethylformamide. Palladium on charcoal (10%; 0.25 g) was added and hydrogen was bubbled through for 30 minutes with stirring. The catalyst was filtered off and 0.42 g of (Z)-2-amino-α-[[2-(diphenylmethoxy)-1,1-dimethyl-2-oxo-ethoxy]imino]-4-thiazoleacetic acid, 0.01 g N-hydroxybenzotriazole and 0.24 g of dicyclohexylcarbodiimide was added; the solution was stirred at ambient temperature for 12 hours. The precipitate was filtered off and the solvent removed *in vacuo*. The residue was suspended in diethyl ether and the precipitate filtered off (0.76 g). This compound was suspended in 3 ml of anisole and cooled to −15°C; 6 ml of trifluoroacetic acid was slowly dropped in with stirring, so that the temperature did not exceed −10°C. After the additon was complete, the temperature was maintained for 2 hours. Cold diethyl ether (100 ml) was added and the precipitate was filtered off, dried and dissolved in water. The pH was adjusted to 6.5 by addition of 1N KOH and the solution was chromatographed on HP—20 resin, using water at eluent, yielding 200 mg of product, melting point 245—250°C, *dec.*

Example 8

[3S(Z)]-2-Amino-N-[1-[[[[(methoxyacetyl)amino]sulfonyl]amino]carbonyl]-2-oxo-3-azetidinyl]-α-(methoxyimino)-4-thiazoleacetamide, potassium salt

(S)-[1-[[[[(Methoxyacetyl)amino]sulfonyl]amino]carbonyl]-2-oxo-3-azetidinyl]carbamic acid, phenylmethyl ester, monopotassium salt (0.5 g; see example 7A) was hydrogenated in 30 ml of dry dimethylformamide in the presence of 0.25 g 10% palladium on charcoal for 30 minutes. The catalyst was filtered off and 0.22 g of (Z)-2-amino-α-(methoxyimino)-4-thiazoleacetic acid, 0.01 g of N-hydroxybenzotriazole and 0.21 g of dicyclohexylcarbodiimide were added. The solution was stirred overnight, filtered and evaporated to dryness. The residue was chromatographed (HP—20, water as eluent), yielding 0.12 g of product, melting point 170—175°C, *dec.*

Example 9

[3S(Z)-2-[[[1-(2-Amino-4-thiazolyl)-2-[[1-[[[(benzoylamino)sulfonyl]amino]carbonyl]-2-oxo-3-azetidinyl]amino]-2-oxoethylidene]amino]oxo]-2-methylpropanoic acid, dipotassium salt

A) (S)-[1-[[(Aminosulfonyl)amino]carbonyl]-2-oxo-3-azetidinyl]carbamic acid, phenylmethyl ester, potassium salt

*Method I*

(S)-(2-Oxo-3-azetidinyl)carbamic acid, phenylmethyl ester (11 g) was dissolved in a mixture of 200 ml of acetonitrile and 50 ml of dichloromethane. The mixture was cooled to −50°C and a solution of chlorosulfonyl isocyanate (9 g) in 25 ml of dichloromethane was added with stirring. After warming the mixture to −30°C a solution of 6 g of ammonia in 60 ml of acetonitrile was added slowly. The reaction temperature was raised to −10°C and finally to 0—5°C. The reaction time was 3 hours. The ammonium salt of the title compound precipitated and was filtered by suction (20 g). The crude product was purified by HP—20 chromatography (0.075—0.150 mm) eluting with 2000 ml of water and water/acetone (8:2); 20 ml fractions were taken. The elution was monitored by thin-layer chromatography. From fractions 142—154, 9.3 g of product was obtained by evaporation.

The ammonium salt of the title compound was dissolved in 100 ml of water, layered with 200 ml of ethyl acetatye and acidified. After separation and washing of the aqueous layer twice with ethyl acetate, the organic layer was washed with saturated sodium chloride solution, dried with anhydrous magnesium sulfate and evaporated to yield 8.1 g of the free acid of the title compound.

*Method II*

A mixture of (S)-(2-oxo-3-azetidinyl)carbamic acid, phenylmethyl ester (11 g) in 175 ml of dichloromethane was cooled to −30°C. While stirring, 7.7 g of chlorosulfonyl isocyanate in 75 ml dichloromethane was added dropwise within 15 minutes. The temperature of the solution was allowed to rise to 0°C over 30 minutes. Subsequently the clear solution was again cooled to −30°C and 8.8 g of bis-(trimethylsilyl)amine dissolved in 30 ml of dichloromethane, was dropped in, while passing dry nitrogen through the flask. After an hour the reaction temperature was allowed to rise to −15°C and was maintained for an additional 30 minutes. The solvent was distilled off *in vacuo*, and the residue was triturated with 400 ml of ether to give a solid (16.6 g) which was washed with an additional 20 ml of ether. From the ethereal mother liquor there was obtained a second crop of 4.2 g of product.

The crude material (18.0 g) along with about 20 g of HP—20 resin was suspended in 30 ml of water and the mixture was chromatographed on an HP—20 column eluted with a) 3 L of water; b) 2.5 L of water/acetone (8:2); 3) 4 L of water/acetone (7:3); 6 L of water/acetone (6:4). Fraction d yielded 6.2 g of the title compound, melting point 150—152°C.

B) (S)-[1-[[[(Benzoylamino)sulfonyl]amino]carbonyl]-2-oxo-3-azetidinyl]carbamic acid, phenylmethyl ester, potassium salt

(S)-[1-[[(Aminosulfonyl)amino]carbonyl]-2-oxo-3-azetidinyl]carbamic acid, phenylmethyl ester,

potassium salt (2.3 g) and 2.2 g of potassium carbonate were stirred in 50 ml of dry dimethylformamide with 5 g of benzoyl chloride and 0.6 g of dimethylaminopyridine overnight. The solvent was removed *in vacuo* and the residue was extracted at pH 1 (aqueous solution) with ethyl acetate. The organic layer was dried and evaporated to dryness. The residue was dissolved in water/acetone (1:9) and the pH adjusted to 6.5 with 1N KOH. The acetone was removed *in vacuo* and the remaining aqueous solution freeze-dried. Purification of the resulting white powder was achieved by HP—20 chromatography using water/acetone (9:1) as eluent, and yielding 1.1 g of product melting point 96—99°C, *dec.*

C) [3S(Z)]-2-[[[1-[2-Amino-4-thiazolyl)-2-[[1-[[[(benzoylamino)sulfonyl]amino]carbonyl]-2-oxo-3-azetidinyl]amino]-2-oxoethylidene]amino]oxy]-2-methylpropanoic acid, dipotassium salt

(S)-[1-[[[(Benzoylamino)sulfonyl]amino]carbonyl]-2-oxo-3-azetidinyl]carbamic acid, phenylmethyl ether, potassium salt (0.5 g) was hydrogenated in 100 ml of dry dimethylformamide in the presence of 0.25 g of 10% palladium on charcoal for 1 hour. The catalyst was filtered off and 0.46 g of (Z)-2-amino-α-[[2-(diphenylmethoxy)-1,1-dimethyl-2-oxoethoxy]imino]-4-thiazoleacetic acid, 0.01 g N-hydroxybenzotriazole and 0.24 g of dicyclohexylcarbodiimide were added and the solution was stirred for 12 hours. The solvent was distilled off *in vacuo* and the residue was filtered off with 100 ml of eher and dried (0.92 g). This compound was suspended in 2 ml of anizole, cooled to −10°C and 4 ml trifluoroacetic acid was added with stirring. The temperature was maintained for 5 hours. After this time 100 ml of dry ether was added and the precipitated compound was filtered off, dissolved in 5 ml of water and the pH was adjusted to 6.5 with 1N KOH. The resulting aqueous solution was chromatographed on HP—20 resin (eluting with water) and yielded 0.24 g of product, melting point 225—230°C, *dec.*

Example 10

[3S(R)]-N-[2-l[[[(Benzoylamino)sulfonyl]amino]carbonyl]-2-oxo-3-azetidinyl]amino]-2-oxo-1-phenylethyl)-4-ethyl-2,3-dioxo-1-piperazine carboxamide, potassium salt

(S)-[1-[[l(Benzoylamino)sulfonyl]amino]carbonyl]-2-oxo-3-azetidinyl]carbamic acid, phenylmethyl ester, potassium salt (0.25 g; see example 9B) was hydrogenated as described in Example 9C. (R)-α-[[(4-Ethyl-2,3-dioxo-1-piperazinyl)carbonyl]amino]benzeneacetic acid (0.17 g), 0./01 g of N-hydroxybenzotriazole and 0.13 g of dicyclohexylcarbodiimide were added to the resulting solution which was stirred overnight. The solvent was distilled off *in vacuo* and the residue chromatographed using HP—20 resin and water/acetone (19:1) as eluent, yielding 0.14 g of product, melting point 180—185°C, *dec.*

Example 11

[3S(Z)]-2-Amino-N-[1-[[[(benzoylamino)sulfonyl]amino]carbonyl]-2-oxo-3-azetidinyl]-α-(methoxyimino)-4-thiazoleacetamide, potassium salt

(S)-[1-[[[(Benzoylamino)sulfonyl]amino]carbonyl]-2-oxo-3-azetidinyl]carbamic acid, phenylmethyl ester, potassium salt (0.25 g; see example 9B) was hydrogenated as described in example 9C. To the resulting solution 0.01 g of N-hydroxybenzotriazole, 0.11 g of (Z)-2-amino-α-(methoxyimino)-4-thiazoleacetic acid and 0.13 g of dicyclohexylcarbodiimide were added. After stirring overnight at room temperature, the solvent was removed *in vacuo* and the residue chromatographed using HP—20 resin and water/acetone (19:1) as eluent, yielding 0.05 g of product, melting point 205—210°C.

Example 12

(S)-[1-[[[[(Acetyl)methylamino]sulfonyl]amino]carbonyl]-2-oxo-3-azetidinyl]carbamic acid, phenylmethyl ester

(S)-3-[[(Phenylmethoxy)carbonyl]amino]-2-azetidinone (2.2 g) was suspended in 100 ml of anhydrous tetrahydrofuran and cooled to −50°C with stirring. Chlorosulfonylisocyanate (1.56 g) was added and the temperature was maintained at 0°C for 30 minutes. After this time 3.51 g of heptamethyl disilazane was added and stirring at room temperature was continued overnight. Acetyl chloride (3.14 g) was added and the solution was stirred for an additional 48 hours. The solvent was removed *in vacuo*, and the residue dissolved in ethyl acetate and extracted twice with 50 ml portions of water. The aqueous layer was discarded and the ethyl acetate solution was dried with $Na_2SO_4$ and evaporated to dryness. The residue was dissolved in acetone/water (9:1), the pH adjusted to 6.5 with 1N KOH and the acetone removed *in vacuo*. The remaining aqueous solution was freeze-dried. The resulting crude compound (1.9 g) was purified by HP—20 chromatography, yielding 1.03 g of product.

Example 13

[3S(Z)]-2-Amino-N-[1-[[[[(2-methylpropanoyl)amino]sulfonyl]amino]carbonyl]-2-oxo-3-azetidinyl]-α-(methoxyimino)-4-thiazoleacetamide potassium salt

(S)-3-[[(Phenylmethoxy)carbonyl]amino]-2-azetidinone (2.2 g) was suspended in 100 ml of dry ethyl acetate, cooled to −50°C and 1.56 g chlorosulfonylisocyanate was added. After 30 minutes at 0°C a clear solution was obtained and 4.78 g of N-trimethylsilyl 2-methylpropionamide was added and stirring at ambient temperature was continued for 12 hours. The solution was washed with 100 ml of water, dried and evaporated to dryness. The residue was dissolved in water/acetone (1:9) and the pH adjusted to 6.5 with 1N KOH. Acetone was removed *in vacuo* and the remaining aqueous solution freeze-dried, yielding 0.28 g of

compound. This compound was dissolved in 20 ml of dry dimethylformamide and hydrogenated with 0.1 g of 10% palladium on charcoal for 30 minutes. The catalyst was filtered off and 0.1 g of (Z)-2-amino-α-(methoxyimino)-4-thiazoleacetic acid, 0.01 g of N-hydroxybenzotriazole and 0.13 g of dicyclohexylcarbodiimide were added and the mixture was stirred overnight at room temperature. The solvent was removed *in vacuo* and the residue chromatographed on HP-20 resin eluting with $H_2O$/acetone (19:1) and yielding 50 mg of product, melting point 185—190°C, *dec.*

Example 14

[3S(Z)]-2-Amino-N-[1-[[[[(aminocarbonyl)amino]sulfonyl]amino]carbonyl]-2-oxo-3-azetidinyl]-α-(methoxyimino)-4-thiazoleacetamide, potassium salt

A) (S)-[1-[[[[(Aminocarbonyl)amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (13.2 g) was suspended in 300 ml of dry tetrahydrofuran at 0°C. Chlorosulfonylisocyanate (11.1 g) dissolved in 80 ml of dry dichloromethane was dropped in with stirring. The temperature was maintained with cooling at 0°C for 30 minutes. Trimethylsilyl urea (10.5 g) was added and stirring was continued at room temperature overnight. The solvent was removed *in vacuo* and 200 ml of methanol was added. The solution was stirred for 30 minutes, the solvent evaporated and ther residue treated with 200 ml of ethyl acetate and 100 ml of water. The organic layer was separated, dried and filtered. After removal of the solvent, the oily residue was dissolved in 100 ml of acetone and 10 ml of water, and the pH was adjusted to 6.5 by addition of 1N potassium hydroxide. After evaporation of the solvent, the title compound remained. Yield 11.0 g; melting point 120—125°C, *dec.*

B) [3S(Z)]-2-Amino-N-[1-[[[[(aminocarbonyl)amino]sulfonyl]amino]carbonyl]-2-oxo-3-azetidinyl]-α-(methoxyimino)-4-thiazoleacetamide, potassium salt

(S)-[1-[[[[(Aminocarbonyl)amino]sulfonyl]amino]carbonyl]-2-oxo-3-azetidinyl]carbamic acid, phenylmethyl ester, potassium salt (1.4 g) was hydrogenated in 100 ml of dry dimethylformamide with 0.7 g of 10% palladium on charcoal as a catalyst. After 30 minutes the hydrogenation was completed, the catalyst filtered off and 0.66 g of (Z)-2-amino-α-(methoxyimino)-4-thiazoleacetic acid and 0.2 g of N-hydroxybenzotriazole were added. Dicyclohexylcarbodiimide (720 mg), dissolved in 100 ml of dry dimethylformamide was slowly dropped in with stirring during a period of 10 hours. Stirring was continued for an additional 12 hours. The mixture was cooled to 0°C, the precipitated urea filtered off, the solvent removed *in vacuo* and the residue chromatographed on HP-20 resin using water as eluent. Yield 900 mg; melting point 205—210°C, *dec.*

Example 15

[3S(Z)]-2-[[[2-[[1-[[[[(Aminocarbonyl)amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt

(S)-[1-[[[[(Aminocarbonyl)amino]sulfonyl]amino]carbonyl]-2-oxo-3-azetidinyl]carbamic acid, phenylmethyl ester, potassium salt (1.98 g; see example 14A) was hydrogenated in the presence of 1 g of 10% palladium on charcoal in 200 ml of dry dimethylformamide for 30 minutes. The catalyst was filtered off and 300 mg of N-hydroxybenzotriazole and 2.5 g of (Z)-2-amino-α-[[2-(diphenylmethoxy)-1,1-dimethyl-2-oxoethoxy]imino]-4-thiazoleacetic acid was added. Dicyclohexylcarbodiimide (1.1 g), dissolved in 100 ml of dimethylformamide was slowly dropped in (10 hours). When the addition is complete, stirring was continued for an additional 10 hours. The solvent was removed *in vacuo* and the solid residue was suspended in 10 ml of anisole and cooled to 0°C. At this temperature 20 ml of trifluoroacetic acid was dropped in with stirring. The temperature was maintained at 0°C with cooling. After 3.5 hours, the reaction was complete and the solution was poured into 200 ml of ether. The precipitate was filtered off, dried and dissolved in 10 ml of water/acetone (1:1), and the pH was adjusted to 6.5 by the addition of 1N potassium hydroxide. The acetone was removed *in vacuo* and the aqueous phase chromatographed on HP-20 resin using water as eluent. Yield 1.5 g; melting point 258°C, *dec.*

Example 16

[3S(R)]-N-[2-[[1-[[[[(Aminocarbonyl)amino]sulfonyl]amino]carbonyl]-2-oxo-3-azetidinyl]amino]-2-oxo-1-phenylethyl]-4-ethyl-2,3-dioxo-1-piperazinecarboxamide, potassium salt

(S)-[1-[[[[(Aminocarbonyl)amino]sulfonyl]amino]carbonyl]-2-oxo-3-azetidinyl]carbamic acid, phenylmethyl ester, potassium salt (1.34 g; see example 14A) was hydrogenated with 0.6 g of 10% palladium on charcoal as catalyst in 100 ml of dry dimethylformamide for 30 minutes. The catalyst was filtered off and 1.23 g of (R)-α-[[(4-ethyl-2,3-dioxo-1-piperazinyl)carbonyl]amino]benzeneacetic acid and 100 mg of N-hydroxybenzotriazole were added. Dicyclohexylcarbodiimide (0.8 g), in 100 ml of dimethylformamide was dropped in with stirring over a period of 10 hours. Stirring was continued for an additional 10 hours. The solvent was removed *in vacuo* and the residue chromatographed on HP-20 resin, using water as eluent. Yield 590 mg; melting point 198°C, *dec.*

Example 17

[3S(Z)]-2-Amino-α-(methoxyimino)-N-[1-[[[[[(methylamino)carbonyl]amino]sulfonyl]amino]carbonyl]-2-oxo-3-azetidinyl]-4-thiazoleacetamide, potassium salt

A) (S)-[1-[[[[[(Methylamino)carbonyl]amino]sulfonyl]amino]carbonyl]-2-oxo-3-azetidinyl]carbamic acid, phenylmethyl ester, potassium salt

(S)-[1-[[(Aminosulfonyl)amino]carbonyl]-2-oxo-3-azetidinyl]carbamic acid, phenylmethyl ester, potassium salt (1 g; see example 9A) was dissolved in 20 ml of dry dimethylformamide. Methylisocyanate (500 mg), was added and the solution was stirred overnight at ambient temperature. The solvent was removed *in vacuo* and the residue was treated with acetone/ether (1:1) and filtered. Yield 0.9 g; melting point 153—160°C, *dec.*

B) [3S(Z)]-2-Amino-α-(methoxyimino)-N-[1-[[[[[(methylamino)carbonyl]amino]sulfonyl]amino]carbonyl]-2-oxo-3-azetidinyl]-4-thiazoleacetamide, potassium salt

(S)-[1-[[[[[(Methylamino)carbonyl]amino]sulfonyl]amino]carbonyl]-2-oxo-3-azetidinyl]carbamic acid, phenylmethyl ester, potassium salt (437 mg) was hydrogenated in 50 ml of dry dimethylformamide in the presence of 200 mg of 10% palladium on charcoal. After 45 minutes the reaction was complete and the catalyst was filtered off. (S)-2-Amino-α-(methoxyimino)-4-thiazoleacetic acid (220 mg), 50 mg of N-hydroxybenzotriazole and 300 mg of dicyclohexylcarbodiimide were added with stirring at ambient temperature. Stirring was continued for 12 hours, the solution cooled to 0°C and the precipitated urea was filtered off. The solvent of the mother liquor was removed *in vacuo* and the residue was chromatographed on HP-20 resin, using water. Yield 305 mg; melting point 220—225°C, *dec.*

Example 18

[3S(R)]-4-Ethyl-N-[2-[[1-[[[[methyl[(methylamino)carbonyl]amino]sulfonyl]amino]carbonyl]-2-oxo-3-azetidinyl]amino]-2-oxo-1-phenylethyl]-2,3-dioxo-1-piperazinecarboxamide, potassium salt

A) (S)-[1-[[[[Methyl[(methylamino)carbonyl]amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (4.4 g) was suspended in 100 ml of dry tetrahydrofuran and the mixture was cooled to −10°C. At this temperature, 1.6 g of chlorosulfonylisocyanate was added and the reaction mixture was allowed to reach room temperature. Trimethylsilyl-N,N′-dimethylurea (5.2 g) were added and stirring at ambient temperature was continued for 12 hours. The solvent was removed *in vacuo* and the residue was dissolved in 100 ml of ethyl acetate. The organic solution was washed twice with 50 ml portions of water, with 50 ml of 2N phosphoric acid and with brine. The organic layer was evaporated to dryness, the residue dissolved in water/acetone (1:1) (50 ml). The pH was adjusted to 6.5 with 1N potassium hydroxide. The acetone was removed *in vacuo* and the aqueous soloution freeze dried to yield 8 g of crude title compound pure enough for further reactions.

B) [3S(R)]-4-Ethyl-N-[2-[[1-[[[[methyl[(methylamino)carbonyl]amino]sulfonyl]amino]carbonyl]-2-oxo-3-azetidinyl]amino]-2-oxo-1-phenylethyl]-2,3-dioxo-1-piperazinecarboxamide, potassium salt

(S)-[1-[[[[Methyl[(methylamino)carbonyl]amino]sulfonyl]amino]carbonyl]-2-oxo-3-azetidinyl]carbamic acid, phenylmethyl ester, potassium salt (900 mg) was hydrogenated in 50 ml of dry dimethylformamide in the presence of 400 mg of 10% palladium on charcoal. The hydrogen uptake ceased after about 30 minutes. The catalyst was filtered off and 700 mg (R)-α-[[(4-ethyl-2,3-dioxo-1-piperazinyl)carbonyl]amino]benzene-acetic acid, 100 mg of N-hydroxybenzotriazole and 600 mg of dicyclohexylcarbodiimide were added with stirring. After 12 hours at ambient temperature, the precipitated urea was filtered off, and the solvent removed *in vacuo*. The residue was chromatographed on HP-20 resin, using water/acetone (9:1) as eluent. Yield 700 mg; melting point 177°C, *dec.*

Example 19

[3S(Z)]-2-[[[1-(2-Amino-4-thiazolyl)-2-[[1-[[[[methyl[(methylamino)carbonyl]amino]sulfonyl]amino]-carbonyl]-2-oxo-3-azetidinyl]amino]-2-oxoethylidene]amino]oxy-2-methylpropanoic acid, dipotassium salt

(S)-[1-[[[[Methyl[(methylamino)carbonyl]amino]sulfonyl]amino]carbonyl]-2-oxo-3-azetidinyl]carbamic acid, phenylmethyl ester, potassium salt (900 mg; see example 18A) was hydrogenated in 50 ml of dry dimethylformamide in the presence of 450 mg of 10% palladium on charcoal for 30 minutes. The catalyst was filtered off and 1 g of (Z)-2-amino-α-[[2-(diphenylmethoxy)-1,1-dimethyl-2-oxoethoxy]imino]-4-thiazoleacetic acid, 100 mg of N-hydroxybenzotriazole and 600 mg of dicyclohexylcarbodiimide were added with stirring at ambient temperature. After 12 hours, the solution was cooled to 0°C and the precipitated urea was filtered off. The solution was evaporated to dryness and the residue was filtered off with 50 ml of ethyl acetate. The crude compound was suspended in 2 ml of anisole, cooled to −5°C and 6 ml of trifluoroacetic acid was dropped in at such a rate that the temperature could be maintained at 0°C. After 2 hours, 100 ml of dry ether was added and the precipitate was filtered off, washed with ether and dried. The crude compound was dissolved in 20 ml of water and the pH adjusted to 6.5 with 1N potassium hydroxide. After freeze drying, the crude potassium salt was chromatographed using HP-20 resin and water as eluent and then freeze-dried. Yield 400 mg; melting point 220°C, *dec.*

Example 20

[3S(Z)]-2-Amino-α-(methoxyimino)-N-[1-[[[[methyl[(methylamino)carbonyl]amino]sulfonyl]amino]-carbonyl]-2-oxo-3-azetidinyl]-4-thiazoleacetamide, potassium salt

(S)-[1-[[[[Methyl[(methylamino)carbonyl]amino]sulfonyl]amino]carbonyl]-2-oxo-3-azetidinyl]carbamic acid, phenylmethyl ester, potassium salt (440 mg; see example 18A) was hydrogenated as described in example 6. (Z)-2-Amino-α-(methoxyimino)-4-thiazoleacetic acid (220 mg), 50 mg of N-hydroxybenzotriazole and 250 ml of dicyclohexylcarbodiimide were added after removal of the catalyst. Stirring at ambient temperature was continued for 12 hours. The solvent was removed *in vacuo* and the residue chromatographed on HP-20 resin, using water/acetone (9:1) as eluent. Yield 350 mg (isolated by freeze drying); melting point 192°C, *dec.*

Example 21

[3S(Z)]-2-[[[1-(2-Amino-4-thiazolyl)-2-[[1-[[[[[(dimethylamino)carbonyl]amino]sulfonyl]amino]carbonyl]-2-oxo-3-azetidinyl]-2-oxoethylidene]amino]oxy]-2-methylpropanoic acid, potassium salt

A) (S)-[1-[[[[(Dimethylamino)carbonyl]amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (4.4 g) was suspended in 150 ml of ethyl acetate and cooled to −15°C. Chorosulfonylisocyanate (3.0 g) was added and the mixture was stirred at 0°C until a clear solution was obtained. N,N-Dimethyurea (2 g) and 2 g of triethylamine were added and stirring was continued overnight. The precipitate was filtered off and the mother liquor washed with two 50 ml portions of water, 50 ml of 2N phosphoric acid and brine. The solvent was removed *in vacuo* and the residue was dissolved in 50 ml of water/acetone (1:1). The pH was adjusted to 6.5 with 1N potassium hydroxide. The acetone was removed *in vacuo* and the aqueous solution was freeze dried. Crude title compound (7 g) was obtained, which was pure enough for further manipulation.

B) [3S(Z)]-2-[[[1-[2-Amino-4-thiazolyl)-2-[[1-[[[[[(dimethylamino)carbonyl]amino]sulfonyl]amino]carbonyl]-2-oxo-3-azetidinyl]amino-2-oxoethylidene]amino]oxy]-2-methylpropanoic acid, potassium salt

(S)-[1-[[[[(Dimethylamino)carbonyl]amino]sulfonyl]amino]carbonyl]-2-oxo-3-azetidinyl]carbamic acid, phenylmethyl ester, potassium salt (451 mg) was dissolved in 50 ml of anhydrous dimethylformamide, 200 ml of 10% palladium on charcoal was added and hydrogen was bubbled through the solution for 45 minutes. The catalyst was filtered off and 450 mg of (Z)-2-amino-α-[[2-(diphenylmethoxy)-1,1-dimethyl-2-oxoethoxy]imino]-4-thiazoleacetic acid, 100 mg of N-hydroxybenzotriazole and 300 mg of dicyclohexylcarbodiimide were added and the solution was stirred at ambient temperature for 12 hours. The precipitated urea was filtered off and the solvent removed *in vacuo*. The residue was chromatographed using HP-20 resin and water/acetone (7:3) as eluent. The benzhydryl ester of the title compound (600 mg) was isolated by freeze drying. This compound was suspended in 2 ml of anisole, the mixture cooled to −10°C and 4 ml of trifluoroacetic acid was slowly dropped in, so that the temperature did not exceed −5°C. When the addition was complete, stirring at −5°C was continued for 2 to 5 hours. The title compound was precipitated by the addition of 100 ml of ether, filtered off, dissolved in 5 ml of water and the pH adjusted to 6.5 with 2N potassium hydroxide. The solution was chromatographed on HP-20 resin using water as eluent. Yield 280 mg; melting point 191°C, *dec.*

Example 22

[3S(Z)-2-[[[1-(2-Amino-4-thiazolyl)-2-[[1-[[[[[(3-ethyl-2-oxo-1-imidazolidinyl)carbonyl]amino]sulfonyl]-amino]carbonyl]-2-oxo-3-azetidinyl]amino]-2-oxoethylidene]amino]oxy]-2-methylpropanoic acid, dipotassium salt

A) (S)-[1-[[[[(3-Ethyl-2-oxo-1-imidazolidinyl)carbonyl]amino]sulfonyl]amino]carbonyl]-2-oxo-3-azetidinyl]-carbamic acid, phenylmethyl ester, potassium salt

(S)-3-[[(Phenylmethoxy)carbonyl]amino]-2-azetidinone (6.6 g) was suspended in 200 ml of anhydrous ethyl acetate and the mixture was cooled to −15°C. At this temperature, 5 g of chlorosulfonylisicyanate was added with stirring at 0°C until a clear solution was obtained (10 minutes). Triethylamine (4 g) and 5.2 g of 1-aminocarbonyl-3-ethylimidazolidin-2-one were added and stirring was continued at room temperature for 12 hours. The reaction mixture was washed with two 50 ml portions of water, 50 ml of 2N phosphoric acid and brine. The organic phase was evaporated to dryness, the residue dissolved in 150 ml of water/acetone (1:1) and the pH was adjusted to 6.5 with 1N potassium hydroxide. The acetone was distilled off *in vacuo* and the remaining aqueous solution was freeze dried. The crude title compound (12.2 g) was pure enough for further reactions.

B) [3S(Z)]-2-[[[1-(2-Amino-4-thiazolyl)-2-[[1-[[[[[(3-ethyl-2-oxo-1-imidazolidinyl)carbonyl]amino]sulfonyl]-amino]carbonyl]-2-oxo-3-azetidinyl]amino]-2-oxoethylidene]amino]oxy]-2-methylpropanoic acid, dipotassium salt

(S)-[1-[[[[(3-Ethyl-2-oxo-1-imidazolidinyl)carbonyl]amino]sulfonyl]amino]carbonyl]-2-oxo-3-azetidinyl]carbamic acid, phenylmethyl ester, potassium salt (520 mg) was hydrogenated in 50 ml of anhydrous dimethylformamide in the presence of 200 mg of 10% palladium on charcoal for 40 minutes. The catalyst was filtered off, 460 mg of (Z)-2-amino-α-[[2-(diphenylmethoxy)-1,1-dimethyl-2-oxoethoxy]imino]-4-thiazoleacetic acid, 100 ml of N-hydroxybenzotriazole and 300 mg of dicyclohexylcarbodiimide were added and the solution was stirred overnight. The solvent was distilled off *in vacuo* and the residue was filtered off with ethyl acetate, suspended in 2 ml of anisole and cooled to −10°C. Trifluoroacetic acid (4 ml) were dropped in at such a rate that the temperature did not exceed −5°C. Stirring at this temperature was continued for 2 hours. After addition of 100 ml ether, the precipitate was filtered off, dissolved in 10 ml of water, the pH adjusted to 6.5 with 1N potassium hydroxide and the aqueous solution was chromatographed on HP-20 using water as eluent, and the title compound isolated by freeze drying. Yield 250 mg; melting point 245°C, *dec.*

Example 23

[3S(Z)]-2-Amino-α-(ethoxyimino)-[1-[[[[[(3-ethyl-2-oxo-1-imidazolidinyl)carbonyl]amino]sulfonyl]amino]carbonyl]-2-oxo-3-azetidinyl]-4-thiazoleacetamide, potassium salt

(S)-[1-[[[[(3-Ethyl-2-oxo-1-imidazolidinyl)carbonyl]amino]sulfonyl]amino]carbonyl]-2-oxo-3-azetidinyl]carbamic acid, phenylmethyl ester, potassium salt (520 mg; see example 22A) was hydrogenated in 50 ml of dimethylformamide in the presence of 200 mg of 10% palladium on charcoal for 40 minutes. The catalyst was filtered off and 230 mg of (Z)-2-amino-α-(ethoxyimino)-4-thiazoleacetic acid, 100 mg of N-hydroxybenzotriazole and 300 mg of dicyclohexylcarbodiimide were added. The solution was agitated at room temperature for 12 hours. After this time the solvent was removed *in vacuo* and the residue filtered with ethyl acetate. Purification of the crude compound was accomplished by HP-20 chromatography using water/acetone (9:1) as eluent, and the title compound isolated by freeze drying. Yield 420 mg; melting point 125°C.

Example 24

[3S(Z)]-2-Amino-N-[1-[[[[[(3-ethyl-2-oxo-1-imidazolidinyl)carbonyl]amino]sulfonyl]amino]carbonyl]-2-oxo-3-azetidinyl]-α-(methoxyimino]-4-thiazoleacetamide, potassium salt

Following the procedure of example 23, but replacing (Z)-2-amino-α-(ethoxyimino)-4-thiazoleacetic acid by (Z)-2-amino-α-(methoxyimino)-4-thiazoleacetic acid, yielded 400 mg of the title compound; melting point 176°C, *dec.*

Example 25

[3S(R)]-4-Ethyl-N-[2-[[1-[[[[[(3-ethyl-2-oxo-1-imidazolidinyl)carbonyl]amino]sulfonyl]amino]carbonyl]-2-oxo-3-azetidinyl]amino]-2-oxo-1-phenylethyl]-2,3-dioxo-1-piperazinecarboxamide, potassium salt

(S)-[1-[[[[(3-Ethyl-2-oxo-1-imidazolidinyl)carbonyl]amino]sulfonyl]amino]carbonyl]-2-oxo-3-azetidinyl]carbamic acid, phenylmethyl ester, potassium salt (520 mg; see example 22A) was hydrogenated in 50 ml of dimethylformamide in the presence of 200 mg of 10% palladium on charcoal for 40 minutes. After filtration, 330 mg of (R)-α-[[(4-ethyl-2,3-dioxo-1-piperazinyl)carbonyl]amino]benzeneacetic acid, 300 mg of dicyclohexylcarbodiimide and 100 mg of N-hydroxybenzotriazole were added and the solution was stirred for 12 hours at room temperature. The solution was evaporated *in vacuo* to dryness and the residue filtered with ethyl acetate. Purification of the crude compound was achieved by chromatography on HP-20 resin using water/acetone (9:1) as eluent. Yield 480 mg; melting point 181°C, *dec.*

Example 26

[3S(Z)]-2-Amino-α-(methoxyimino)-N-[1-[[[[[(4-ethyl-2,3-dioxo-1-piperazinyl)carbonyl]amino]sulfonyl]amino]carbonyl]-2-oxo-3-azetidinyl]-4-thiazoleacetamide, potassium salt

A) (S)-[1-[[[[[(4-Ethyl-2,3-dioxo-1-piperazinyl)carbonyl]amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidine (4.4 g) was suspended in 200 ml of ethyl acetate and at −15°C, 3 g of chlorosulfonylisocyanate was added with stirring. After 15 minutes at 0°C a clear solution was obtained. 2.5 g Triethylamine and 4 g of 1-aminocarbonyl-4-ethyl-2,3-dioxopiperazine was added and stirring was continued for 16 hours. The mixture was washed with two 50 ml portions of water, 50 ml of 2N phosphoric acid and brine. After evaporation of the solvent, the residue was dissolved in 100 ml of acetone/water (1:1), the pH adjusted to 6.5, the acetone removed and 8 g of crude compound isolated by freeze drying.

B) [3S(S)]-2-Amino-α-(methoxyimino)-N-[1-[[[[[(4-ethyl-2,3-dioxo-1-piperazinyl)carbonyl]amino]sulfonyl]amino]carbonyl]-2-oxo-3-azetidinyl]-4-thiazoleacetamide, potassium salt

(S)-[1-[[[[[(4-Ethyl-2,3-dioxo-1-piperazinyl)carbonyl]amino]sulfonyl]amino]carbonyl]-2-oxo-3-azetidinyl]carbamic acid, phenylmethyl ester, potassium salt (548 mg) was hydrogenated in 50 ml of dimethylformamide in the presence of 270 mg of 10% palladium on charcoal for 45 minutes. To the hydrogenation solution, 210 mg of (Z)-2-amino-α-(methoxyimino)-4-thiazoleacetic acid, 50 mg of N-hydroxybenzotriazole and 300 mg of dicyclohexylcarbodiimide were added and the solution was stirred at 20°C for 16 hours. The solvent was removed *in vacuo*, the residue filtered with ethyl acetate and the crude compound was chromatographed on HP-20 resin using water/acetone (9:1) as eluent. Yield 280 mg.

## Example 27

[3S(Z)]-2-[[[2-[[1-[[[[(4-Ethyl-2,3-dioxo-1-piperazinyl)carbonyl]amino]sulfonyl]amino]carbonyl]-2-oxo-3-azetidinyl]-1-(2-amino-4-thiazolyl)-2-oxoethylidene]amino]oxy]-2-methylpropanoic acid, dipotassium salt

(S)-[1-[[[[[(4-Ethyl-2,3-dioxo-1-piperazinyl)carbonyl]amino]sulfonyl]amino]carbonyl]-2-oxo-3-azetidinyl]carbamic acid, phenylmethyl ester, potassium salt (548 mg; see example 26A) was hydrogenated in 50 ml of dimethylformamide in the presence of 270 mg of 10% palladium on charcoal for 45 minutes. After removal of the catalyst, 450 mg of (Z)-2-amino-α-[[2-(diphenylmethoxy)-1,1-dimethyl-2-oxoethoxy]imino]-4-thiazoleacetic acid, 100 mg of N-hydroxybenzotriazole and 300 mg of dicyclohexylcarbodiimide were added. The solution was stirred for 16 hours. The solvent was removed *in vacuo* and the residue was filtered with ethyl acetate. The crude benzhydryl ester of the title compound was suspended in 2 ml of anisole, cooled to −0°C and 4 ml of trifluoroacetic acid was dropped in with stirring. The temperature was maintained at −5°C for 2 hours. Ether (100 ml) was added and the precipitate was filtered off, dried and dissolved in 5 ml of water, the pH adjusted to 6.5 and the solution chromatographed on HP-20 resin using water as eluent. The title compound (180 mg) was isolated by freeze drying.

## Example 28

[3S(R)]-4-Ethyl-N-[2-[[1-[[[[(methylamino)carbonyl]amino]sulfonyl]amino]carbonyl]-2-oxo-3-azetidinyl]amino]-2-oxo-1-phenylethyl-2,3-dioxo-1-piperazinecarboxamide, potassium salt

(S)-[1-[[[[[(Methylamino)carbonyl]amino]sulfonyl]amino]carbonyl]-2-oxo-3-azetidinyl]carbamic acid, phenylmethyl ester, potassium salt (437 mg; see example 17A) was hydrogenated in 50 ml of dry dimethylformamide in the presence of 200 mg of 10% palladium on charcoal for 45 minutes. The catalyst was filtered off, 350 mg of (R)-α-[[(4-ethyl-2,3-dioxo-1-piperazinyl)carbonyl]amino]benzeneacetic acid, 50 mg of N-hydroxybenzotriazole and 300 mg of dicyclohexylcarbodiimide were added; the solution was stirred for 12 hours. After removal of the solvent *in vacuo*, the residue was chromatographed on HP-20 resin using water/acetone (9:1) as eluent. Yield 380 mg; melting point 178—1283°C, *dec.*

## Example 29

[3S(Z)]-2-[[[1-(2-Amino-4-thiazolyl)-2-[[1-[[[[[(methylamino)carbonyl]amino]sulfonyl]amino]carbonyl]-2-oxo-3-azetidinyl]amino]-2-oxoethylidene]amino]oxy]-2-methylpropanoic acid, dipotassium salt

(S)--[1-[[[[[(Methylamino)carbonyl]amino]sulfonyl]amino]carbonyl]-2-oxo-3-azetidinyl]carbamic acid, phenylmethyl ester, potassium salt (437 mg; see example 17A) was hydrogenated in 50 ml of dimethylformamide in the presence of 200 mg of 10% palladium on charcoal for 45 minutes. The catalyst was filtered off and 450 mg of (Z)-2-amino-α-[[2-(diphenylmethoxy)-1,1-dimethyl-2-oxoethoxy]imino]-4-thiazoleacetic acid, 50 mg of N-hydroxybenzotriazole and 300 mg of dicyclohexylcarbodiimide were added. The solution was stirred overnight, evaporated to dryness, the residue suspended in 2 ml of anisole, cooled to −10°C and 4.ml of trifluoroacetic acid was dropped in. Stirring was continued for 2 hours at −5°C. After addition of 100 ml of ether, the precipitate was filtered off, dissolved in 5 ml of water, the pH adjusted to 6.5 with 1N potassium hydroxide and the aqueous solution chromatographed on HP-20 resin using water as eluent. The title compound (280 mg) was isolated by freeze drying, melting point 215—220°C, *dec.*

## Example 30

[3S(Z)]-2-Amino-N-[1-[[[[[(dimethylamino)carbonyl]amino]sulfonyl]amino]carbonyl]-2-oxo-3-azetidinyl]-α-(methoxyimino)-4-thiazolacetamide, potassium salt

(S)-[1-[[[[[(Dimethylamino)carbonyl]amino]sulfonyl]amino]carbonyl]-2-oxo-3-azetidinyl]carbamic acid, phenylmethyl ester, potassium salt (451 mg; see example 21A) was hydrogenated in 50 ml of dimethylformamide in the presence of 200 mg of 10% palladium on charcoal for 45 minutes. The catalyst was filtered off and 201 mg of (Z)-2-amino-α-(methoxyimino)-4-thiazoleacetic acid, 50 mg of N-hydroxybenzotriazole and 300 mg of dicyclohexylcarbodiimide were added. The solution was stirred for 12 hours at ambient temperature. The solvent was removed *in vacuo* and the crystalline residue chromatographed on HP-20 resin using water/acetone (9.5:0.5) as eluent. Yield 285 mg; melting point 201°C, *dec.*

## Example 31

[3S(Z)]-2-Amino-α-(methoxyimino)-N-[2-oxo-1-[[[[[(2-oxo-1-pyrrolidinyl)carbonyl]amino]sulfonyl]amino]carbonyl]-3-azetidinyl]-4-thiazoleacetamide, potassium salt

A) (S)-[2-Oxo-1-[[[[[(2-oxo-1-pyrrolidinyl)carbonyl]amino]sulfonyl]amino]carbonyl]-3-azetidinyl]carbamic acid, phenylmethyl ester, potassium salt

(S)-3-[[(Phenylmethoxy)carbonyl]amino]-2-azetidinone (3 g) was suspended in 100 ml of dry tetrahydrofuran at 0°C. Chlorosulfonylisocyanate (2.1 g) was slowly dropped in with stirring while the temperature was maintained at 0°C; after 30 minutes, a clear solution was obtained. At 0°C, 1.7 g of triethylamine and 2.05 g of N-carbamoylpyrrolidone were added. Stirring was continued at ambient temperature for 15 hours. After this time the precipitate was filtered off and the mother liquor evaporated to dryness. The residue was dissolved in 50 ml of water/acetone (1:1), the pH adjusted to 6.5 with 1N potassium hydroxide, the acetone removed *in vacuo* and the remaining aqueous solution freeze dried yielding 6 of the title compound.

B) [3S(Z)]-2-Amino-α-(methoxyimino)-N-[2-oxo-1-[[[[(2-oxo-1-pyrrolidinyl)carbonyl]amino]sulfonyl]-amino]carbonyl]-3-azetidinyl]-4-thiazoleacetamide, potassium salt

(S)-[2-Oxo-1-[[[[(2-oxo-1-pyrrodinyl)carbonyl]amino]sulfonyl]amino]carbonyl]-3-azetidinyl]carbamic acid, phenylmethyl ester, potassium salt (1 g) was hydrogenated in dimethylformamide using a 10% palladium on charcoal catalyst. The catalyst was filtered off, and 0.44 g of (Z)-2-amino-α-(methoxyimino)-4-thiazoleacetic acid, 30 mg N-hydroxybenzotriazole and 0.82 g dicyclohexylcarbodiimide were added to the mother liquor. The solution was stirred overnight, the solvent removed *in vacuo* and the residue chromatographed on HP-20 resin (eluting with water) yielding 300 mg of the title compound.

Example 32

[3S(Z)]-2-[[[1-(2-Amino-4-thiazolyl)-2-oxo-2-[[2-oxo-1-[[[[(2-oxo-1-pyrroldinyl)carbonyl]amino]sulfonyl]-amino]carbonyl]-3-azetidinyl]amino]ethylidene]amino]oxy]-2-methylpropanoic acid, dipotassium salt

(S)-[2-Oxo-1-[[[[(2-oxo-1-pyrrolidinyl)carbonyl]amino]sulfonyl]amino]carbonyl]-3-azetidinyl]carbamic acid, phenylmethyl ester, potassium salt (1.5 g; see example 31A) was hydrogenated in dimethylformamide using a 10% palladium on charcoal catalyst. The catalyst was filtered off, and 1.47 g of (Z)-2-amino-α-[[2-(diphenylmethoxy)-1,1-dimethyl-2-oxoethoxy]imino]-4-thiazoleacetic acid, 40 mg of N-hydroxybenzotriazole and 1.26 g of dicyclohexylcarbodiimide were added to the mother liquor. The solution was stirred overnight, the solvent removed *in vacuo* and the residue filtered with ether. The residue was suspended in 5 ml of anisole and cooled to −5°C. Trifluoroacetic acid (13 ml) was slowly dropped in with stirring, and after 30 minutes the crude product was precipitated by the addition of 100 ml of ether and filtered off. The product was dissolved in 5 ml of water and the pH adjusted to 6.5 with 1N potassium hydroxide. Chromatography on HP-20 resin (eluting with water) yielded 500 mg of the title compound, melting point 233°C, *dec.*

Example 33

[3S(R)]-4-Ethyl-2,3-dioxo-N-[2-oxo-2-[[2-oxo-1-[[[[(2-oxo-1-pyrrolidinyl)carbonyl]amino]sulfonyl]amino]-carbonyl]-3-azetidinyl]amino]-1-phenylethyl]-1-piperazinecarboxamide, potassium salt

(S)-[2-Oxo-1-[[[[(2-oxo-1-pyrroldinyl)carbonyl]amino]sulfonyl]amino]carbonyl]-3-azetidinyl]carbamic acid, phenylmethyl ester, potassium salt (1 g; see example 31A) was hydrogenated in dimethylformamide using a 10% palladium on charcoal catalyst. The catalyst was filtered off, 0.7 g of (R)-α-[[(4-ethyl-2,3-dioxo-1-piperazinyl)carbonyl]amino]benzeneacetic acid, 30 mg of N-hydroxybenzotriazole and 0.82 g of dicyclohexylcarbodiimide were added to the mother liquor, and the mixture was stirred overnight at ambient temperature. The solvent was removed *in vacuo* and the residue chromatographed on HP-20 resin, eluting with water, to yield 300 mg of the title compound, melting point 186°C.

Example 34

[3S(Z)]-2-Amino-α-(methoxyimino)-N-[2-oxo-1-[[[[(2-oxo-3-oxazolidinyl)carbonyl]-amino]sulfonyl]amino]carbonyl]-3-azetidinyl]-4-thiazoleacetamide, potassium salt

A) (S)-[2-Oxo-1-[[[[(2-oxo-3-oxazolidinyl)-carbonyl]amino]sulfonyl]amino]carbonyl]-3-azetidinyl]carbamic acid, phenylmethyl ester, potassium salt

(S)-3-[[(Phenylmethoxy)carbonyl]amino]-2-azetidinone (3.0 g) was suspended in 300 ml of dry tetrahydrofuran and cooled to −70°C. Chlorosulfonylisocyanate (2.3 g) dissolved in 10 ml of dry tetrahydrofuran was dropped in with stirring. The temperature was allowed to rise to 0°C and this temperature was maintained for 10 minutes. Afterwards it was cooled to −40°C. Triethylamine (1.65 g) and 1.95 g of N-carbamoyl-2-oxooxazolidine were added. The mixture was stirred at room temperature for 14 hours and the solvent was removed *in vacuo*. After dissolution in ethyl acetate, 100 ml of 2N phosphoric acid was added and extracted three times with ethyl acetate. The combined organic layers were evaporated *in vacuo*, the residue was dissolved in 100 ml of acetone/water (1:1) and the pH was adjusted to 6.5 with 1N potassium hydroxide. After removal of the acetone *in vacuo*, the aqueous solution was freeze dried yielding 5.2 g of crude product.

B) [3S(Z)]-2-Amino-α-(methoxyimino)-N-[2-oxo-1-[[[[(2-oxo-3-oxazolidinyl)carbonyl]amino]sulfonyl]-amino]carbonyl]-3-azetidinyl]-4-thiazoleacetamide, potassium salt

(S)-[2-Oxo-1-[[[[(2-oxo-3-oxazolidinyl)-carbonyl]amino]sulfonyl]amino]carbonyl]-3-azetidinyl]-carbamic acid, phenylmethyl ester, potassium salt (1 g) was hydrogenated in 80 ml of dry dimethylformamide using 0.5 g of 10% palladium on charcoal as catalyst. After 20 minutes, the hydrogenation was completed and the catalyst was filtered off. (Z)-2-Amino-α-(methoxyimino)-4-thiazoleacetic acid (0.44 g), 30 mg of N-hydroxybenzotriazole and 0.91 g of dicyclohexylcarbodiimide were added to the mother liquor. The solution was stirred for 12 hours at room temperature, the solvent removed *in vacuo* and the 1.7 g residue was chromatographed using HP-20 resin and water as eluent, yielding 0.3 g of the title compound, melting point 208°C, *dec.*

| | | | | | |
|---|---|---|---|---|---|
| Analysis for $C_{14}H_{15}KN_8O_9S_2$: | C: 30.99; | H: 2.79; | N: 20.65; | S: 11.82; | K: 7.21. |
| Found: | C: 30.08; | H: 3.13; | N: 19.52; | S: 10.34; | K: 7.41 |

Example 35

[3S(R)]-4-Ethyl-2,3-dioxo-N-[2-oxo-2-[[2-oxo-1-[[[[(2-oxo-3-oxazolidinyl)carbonyl]amino]sulfonyl]amino]-carbonyl]-3-azetidinyl]amino]-1-phenylethyl]-1-piperazinecarboxamide, potassium salt

(S)-[2-Oxo-1-[[[[(2-oxo-3-oxazolidinyl)carbonyl]amino]sulfonyl]amino]carbonyl]-3-azetidinyl]carbamic acid, phenylmethyl ester, potassium salt (1 g; see example 34A) was hydrogenated in 80 ml of dry dimethylformamide using 0.5 g of a 10% palladium on charcoal catalyst. The catalyst was filtered off, 0.7 g of (R)-α-[[(4-ethyl-2,3-dioxo-1-piperazinyl)carbonyl]amino]benzeneacetic acid, 30 mg of N-hydroxybenzotriazole and 0.91 g of dicyclohexylcarbodiimide were added to the mother liquor, and the mixture was stirred for 14 hours at ambient temperature. The solvent was removed *in vacuo* and the residue chromatographed on HP-20 resin, eluting with water, to yield 0.36 g of the title compound, melting point 187°C, *dec.*

| | | | | | |
|---|---|---|---|---|---|
| Analysis for $C_{23}H_{25}KN_8O_{11}S$: | C: 41.81; | H: 3.81; | N: 16.96; | S: 4.85; | K: 5.92 |
| Found: | C: 40.04; | H: 3.93; | N: 16.33; | S: 4.45; | K: 5.59 |

Example 36

[3S(Z)]-2-[[[1-(2-Amino-4-thiazolyl)-2-oxo-2-[[2-oxo-1-[[[[(2-oxo-3-oxazolidinyl)carbonyl]amino]sulfonyl]-amino]carbonyl]-3-azetidinyl]amino]ethylidene]amino]oxy]-2-methylpropanoic acid, dipotassium salt

(S)-[2-Oxo-1-[[[[(2-oxo-3-oxazolidinyl)carbonyl]amino]sulfonyl]amino]carbonyl]-3-azetidinyl]carbamic acid, phenylmethyl ester, potassium salt (1 g; see example 34A) was hydrogenated in 80 ml of dimethylformamide using 0.5 g of a 10% palladium on charcoal catalyst. The catalyst was filtered off, and 0.97 g of (z)-2-amino-α-[[2-(diphenylmethoxy)-1,1-dimethyl-2-oxoethoxy)imino]-4-thiazolacetic acid, 30 mg of N-hydroxybenzotriazole and 0.91 g of dicyclohexylcarbodiimide were added to the mother liquor. The solution was stirred for 14 hours and the solvent removed *in vacuo*. The residue was suspended in 4 ml of anisole and cooled to −5°C. Trifluoroacetic acid (12 ml) was slowly dropped in with stirring, and after 20 minutes the crude product was precipitated by the addition of 100 ml of ether and filtered off. The product was dissolved in 20 ml of water/acetone (1:1) and the pH adjusted to 6.5 with 1N potassium hydroxide. Acetone was evaporated, the residue was freeze-dried and the crude product was chromatographed on HP-20 resin to yield 0.28 g of the title compound, melting point 250°C, *dec.*

| | | | | | |
|---|---|---|---|---|---|
| Analysis for $C_{17}H_{18}K_2N_8O_{11}S_2$: | C: 31.28; | H, 2.78; | N: 17.17; | S: 9.83; | K: 11.98 |
| Found: | C: 29.54; | H: 3.23; | N: 16.93; | S: 8.97; | K: 11.86 |

[3S(Z)]-2-Amino-N-[1-[[[[3-(aminoethyl)-2-oxo-1-imidazolidinyl]carbonyl]amino]sulfonyl]amino]carbonyl]-2-oxo-3-azetidinyl]-α-(methoxyimino)-4-thiazoleacetamide, potassium salt

(S)-3-[[(Phenylmethoxy)carbonyl]amino]-2-azetidinone (2 g) was suspended in 200 ml of dry tetrahydrofuran and cooled to −70°C. Chlorosulfonylisocyanate (1.4 g) dissolved in 7 ml of dry tetrahydrofuran was added with stirring. The temperature was allowed to rise to 0°C and this temperature was maintained for 10 minutes. Afterwards it was cooled to −40°C, 1.2 g of triethylamine was dropped in and 2.9 g of 1-[2-[(*t*-butoxycarbonyl)amino]ethyl]3-carbmoyl-2-oxoimidazoline was added. This mixture was stirred at room temperature for 14 hours. Following the work-up procedure described in example 34A yielded 5.4 g of (S)-[1-[[[[(3-[2-(*t*-butoxycarbonylamino)ethyl]-2-oxo-1-imidazoldinyl]carbonyl]amino]-sulfonyl]amino]carbonyl]-2-oxo-3-azetidinyl]carbamic acid, phenylmethyl ester, potassium salt.

The above-prepared compound (2.7 g) was hydrogenated in 250 ml of dry dimethylformamide using 1.4 g of 10% palladium on charcoal catalyst. After 30 minutes, the hydrogenation was completed and the catalyst was filtered off. (Z)-2-Amino-α-(methoxyimino)-4-thiazoleacetic acid (0.95 g), 54 mg of N-hydroxybenzotriazole and 1.75 g of dicyclohexylcarbodiimide were added to the mother liquor. The solution was stirred for 12 hours at room temperature, the solvent removed *in vacuo* and the residue dissolved in 6 ml of anisole and cooled to −5°C. Trifluoroacetic acid (15 ml) was carefully dropped in with stirring. The temperature was maintained for an additional 3 hours, and the title compound precipitated by the addition of 150 ml of dry ether. The product was filtered and dissolved in 30 ml of water/acetone (1:1), and the pH was adjusted to 6.5 by the addition of 1N potassium hydroxide. Acetone was evaporated, the residue freeze-dried and 2.7 g of crude product was chromatographed on HP-20 resin eluting with water, to yield 0.25 g of the title compound, melting point 193°C, *dec.*

Example 38

[3S(Z)]-2-[[[2-[[1-[[[[[3-(2-Aminoethyl)-2-oxo-1-imidazolidinyl]carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt

(S)-[1-[[[[[3-[2-(*t*-Butoxycarbonylamino)ethyl]-2-oxo-1-imidazolidinyl]carbonyl]amino)-ethyl]-2-oxo-1-imidazolidinyl]carbonyl]amino]sulfonyl]amino]carbonyl]-2-oxo-3-azetidinyl]carbamic acid, phenylmethyl ester, potassium salt (2.7 g; see example 37) was hydrogenated in dry dimethylformamide using 10% palladium on charcoal as a catalyst. The catalyst was filtered off, and (Z)-2-amino-α-[[2-(diphenylmethoxy)-1,1-dimethyl-2-oxoethoxy]imino]-4-thiazoleacetic acid (2.1 g), 54 mg of N-hydroxybenzotriazole and 1.75 g of dicyclohexylcarbodiimide were added to the mother liquor. Following the work-up procedure described

in example 37 and chromatography on HP-20 resin eluting with water yielded 0.3 g of the title compound, melting point 221°C, *dec.*

Example 39

[3S(Z)]-2-Amino-N-[1-[[[[[3-(2-hydroxyethyl)-2-oxo-1-imidazolidinyl]carbonyl]amino]sulfonyl]amino]carbonyl]-2-oxo-3-azetidinyl]-α-(methoxyimino]-4-thiazoleacetamide, potassium salt

(S)-3-[[(Phenylmethoxy)carbonyl]amino]-2-azetidinone (1.5 g) was suspended in 100 ml of dry tetrahydrofuran and cooled to −70°C. Chlorosulfonylisocyanate (1.1 g) dissolved in 10 ml of dry tetrahydrofuran was dropped in with stirring. The temperature was allowed to rise to 0°C and maintained there for 20 minutes, followed by cooling to −40°C. Triethylamine (0.9 g) and 2.8 g of 1-carbamoyl-2-oxo-3-[2-[(triphenylmethyl)oxy]ethyl]imidazolidine were added and the mixture was stirred at room temperature for 14 hours. The solvent was removed *in vacuo* and the work-up procedure of example 34A was followed yielding 4.3 g of (S)-[1-[[[[[3-(2-hydroxyethyl)-2-oxo-1-imidazolidinyl]carbonyl]amino]sulfonyl]amino]-carbonyl]-2-oxo-3-azetidinyl]carbamic acid, phenylmethyl ester, potassium salt.

The above-prepared compound was hydrogenated in 80 ml of dry dimethylformamide using 0.3 g of 10% palladium on charcoal catalyst. After 30 minutes the hydrogenation was completed and the catalyst was filtered off. (Z)-2-Amino-α-(methoxyimino)-4-thiazoleacetic acid (195 mg), 13 mg of N-hydroxybenzotriazole and 360 mg of dicyclohexylcarbodiimide were added to the mother liquor. The solution was stirred for 12 hours at room temperature, the solvent removed *in vacuo* and the residue precipitated with ether. Crude product (0.5 g) was purified on HP-20 resin (eluent water/acetone (9.5:0.5)) yielding 30 mg of the title compound, melting point 193°C, *dec.*

| | | | | | |
|---|---|---|---|---|---|
| Analysis for $C_{16}H_{19}KN_9O_9S_2$: | C: 32.87; | H: 3.28; | N: 21.56; | S: 10.97; | K: 6.69 |
| Found: | C: 31.26; | H: 3.44; | N: 18.56; | S: 8.86; | K: 6.56 |

Example 40

[3S(Z)]-2-[[[1-(2-Amino-4-thiazolyl)-2-[[1-[[[[[3-(2-hydroxyethyl)-2-oxo-1-imidazolidinyl]carbonyl]amino]sulfonyl]amino]carbonyl]-2-oxo-3-azetidinyl]amino]-2-oxoethylidene]amino]oxy]-2-methylpropanoic acid, dipotassium salt

(S)-[1-[[[[[3-(2-Hydroxyethyl)-2-oxo-1-imidazolidinyl)]carbonyl]amino]sulfonyl]amino]carbonyl]-2-oxo-3-azetidinyl]carbamic acid, phenylmethyl ester, potassium salt (470 mg; see example 39) was hydrogenated in 80 ml of dry dimethylformamide using 0.3 g of 10% palladium on charcoal as a catalyst. After 30 minutes the catalyst was filtered off, and (Z)-2-amino-α-[[2-(diphenylmethoxy)-1,1-dimethyl-2-oxoethoxy]imino]-4-thiazoleacetic acid, 13 mg of N-hydroxybenzotriazole and 360 mg of dicyclohexylcarbodiimide were added to the mother liquor. Following the procedure described in example 36 yielded the title compound, melting point 219°C, *dec.*

Example 41

[3S(Z)]-2-[[[2-[[1-[[[[(3-Amino-2-oxo-1-imidazolidinyl)carbonyl]amino]sulfonyl]amino]carbonyl]-2-oxo-3-azetidinyl]amino)-1-(2-amino-4-thiazolyl)-2-oxoethylidene]amino]oxy]-2-methylpropanoic acid, dipotassium salt

A) (S)-[1-[[[[[3-[(*t*-Butoxycarbonyl)amino]-2-oxo-1-imidazolidinyl]carbonyl]-amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (1.5 g) were suspended in 150 ml of dry tetrahydrofuran and cooled to −60°C. Chlorosulfonylisocyanate (1.1 g) dissolved in 20 ml of dry tetrahydrofuran was dropped in with stirring. The temperature was allowed to rise to 0°C and this temperature was maintained for 15 minutes and then cooled to −60°C. Triethylamine (0.9 g) and 1.7 g of 1-carbamoyl-3-[(*t*-butoxycarbonyl)-amino]-2-oxoimidazolidine were added. The mixture was stirred at room temperature for 14 hours and then worked-up using the procedure described in example 34, part A, yielding 1.5 g of the title compound.

B) [3S(Z)]-2-[[[2-[[1-[[[[(3-Amino-2-oxo-1-imidazoldinyl)carbonyl]amino]sulfonyl]amino]carbonyl]-2-oxo-3-azetidinyl]amino-1-(2-amino-4-thiazolyl)-2-oxoethylidene]amino]oxy]-2-methylpropanoic acid, dipotassium salt

(S)-[1-[[[[[3-[(*t*-Butoxycarbonyl)amino]-2-oxo-1-imidazolidinyl]carbonyl]amino]sulfonyl]amino]carbonyl]-2-oxo-3-azetidinyl]carbamic acid, phenylmethyl ester, potassium salt (0.79 g) was hydrogenated in 60 ml of dry dimethylformamide using 0.4 g of 10% palladium on charcoal catalyst. After 20 minutes the hydrogenation was completed and the catalyst was filtered off. (Z)-2-Amino-α-[[2-(diphenylmethoxy)-1,1-dimethyl-2-oxoethoxy]imino]-4-thiazoleacetic acid (0.63 g), 19 mg of N-hydroxybenzotriazole and 540 mg of dicyclohexylcarbodiimide were added to the mother liquor. Following the procedure described in example 34B, yielded 48 mg of the title compound, melting point 208°C, *dec.*

Example 42

[3S(Z)]-2-Amino-N-[1-[[[[[(3-methyl-2-oxo-1-imidazolidinyl)carbonyl]amino]sulfonyl]amino]carbonyl]-2-oxo-3-azetidinyl]-α-(methoxyimino)-4-thiazoleacetamide, potassium salt

A) (S)-[1-[[[[[(3-Methyl-2-oxo-1-imidazolidinyl)-carbonyl]amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (3.0 g) was suspended in 300 ml of dry tetrahydrofuran and cooled to −70°C. Chlorosulfonylisicyanate (2.1 g) dissolved in 10 ml of dry tetrahydrofuran was dropped in with stirring. The temperature was allowed to rise to 0°C and this temperature was maintained for 10 minutes, then cooled to −40°C. Triethylamine (1.7 g) and 2.3 g of 1-carbamoyl-3-methyl-2-oxoimidazoldine were added. Following the procedure described in example 34A yielded 4.3 g of the title compound.

B) [3S(Z)]-2-Amino-N-[1-[[[[[(3-methyl-2-oxo-1-imidazolidinyl)carbonyl]amino]sulfonyl]amino]carbonyl]-2-oxo-3-azetidinyl]-α-(methoxyimino)-4-thiazoleacetamide, potassium salt

(S)-[1-[[[[[(3-Methyl-2-oxo-1-imidazolidinyl)carbonyl]amino]sulfonyl]amino]carbonyl]-2-oxo-3-azetidinyl]carbamic acid, phenylmethyl ester, potassium salt (1.2 g) was hydrognated in 80 ml of dry dimethylformamide using 0.6 g of 10% palladium on charcoal as catalyst. After 30 minutes the catalyst was filtered off, and (Z)-2-amino-α-(methoxyimino)-4-thiazoleacetic acid (0.52 g), 32 mg of N-hydroxybenzotriazole and 1.0 g of dicyclohexylcarbodiimide were added to the mother liquor. The solution was stirred for 13 hours at room temperature and the solvent was then removed *in vacuo*. The residue was chromatographed on HP-20 resin, eluting with water, to yield 0.120 g of the title compound, melting point 205°C, *dec.*

| | | | | | |
|---|---|---|---|---|---|
| Analysis for $C_{15}H_{18}KN_9O_8S_2$: | C: 32.43; | H: 3.27; | N: 22.69; | S: 11.54; | K: 7.04 |
| Found: | C: 30.83; | H: 3.44; | N: 21.34; | S: 10.20; | K: 7.30 |

Example 43

[3S(Z)]-2-[[[2-[[1-[[[[[(3-Methyl-2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt

(S)-[1-[[[[[(3-Methyl-2-oxo-1-imidazolidinyl)carbonyl]amino]sulfonyl]amino]carbonyl]-2-oxo-3-azetidinyl]carbamic acid, phenylmethyl ester, potassium salt (1.5 g; see example 42A) was hydrogenated in 80 ml of dry dimethylformamide using 0.8 g of 10% palladium on charcoal as catalyst. After 30 minutes the catalyst was filtered off and 1.4 g of (Z)-2-amino-α-[[2-(diphenylmethoxy)-1,1-dimethyl-2-oxoethoxy]-imino]-4-thiazoleacetic acid, 40 mg of N-hydroxybenzotriazole and 1.3 g of dicyclohexylcarbodiimide were added to the mother liquor. Following the procedure of example 36 yielded 0.5 g of the title compound, melting point 211°C, *dec.*

| | | | | | |
|---|---|---|---|---|---|
| Analysis for $C_{18}H_{21}K_2N_9O_{10}S_2$: | C: 32.47; | H: 3.18; | N: 18.94; | S: 9.63; | K: 11.75 |
| Found: | C: 30.55; | H: 3.53; | N: 18.12; | S: 8.65; | K: 10.97 |

Example 44

[3S(Z)]-2-[[[2-[[1-[[[[[(2-Oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt

A) (S)-[1-[[[[[[2-Oxo-3-(triphenylmethyl)-1-imidazolidinyl]carbonyl]amino]sulfonyl]amino]carbonyl]-2-oxo-3-azetidinyl]carbamic acid, phenylmethyl ester, potassium salt

(S)-3-[[(Phenylmethoxy)carbonyl)amino]-2-azetidinone (1.3 g) was suspended in 130 ml of dry tetrahydrofuran and cooled to −70°C. Chlorosulfonylisocyanate (0.9 g) dissolved in 5 ml of dry tetrahydrofuran was dropped in with stirring. The temperature was allowed to rise to 0°C and maintained there for 10 minutes, followed by cooling to −40°C. Triethylamine (0.8 g) and 0.65 g of 1-carbamoyl-2-oxo-3-(triphenylmethyl)imidazolidine were added and the mixture was worked-up following the procedure described in example 34A to yield 3.2 g of the title compound.

B) [3S(S)]-2-[[2-[[2-[[1-[[[[[(2-Oxo-1-imidazolidinyl)carbonyl]amino]sulfonyl]amino]carbonyl]-2-oxo-3-azetidinyl]amino]-1-(2-amino-4-thiazolyl)-2-oxo-ethylidene]amino]oxy]-2-methylpropanoic acid, dipotassium salt

(S)-[1-[[[[[[2-Oxo-3-(triphenylmethyl)-1-imidazolidinyl]carbonyl]amino]sulfonyl]amino]carbonyl]-2-oxo-3-azetidinyl]carbamic acid, phenylmethyl ester, potassium salt (1.5 g) was hydrogenated in 80 ml of dry dimethylformamide using 0.9 g of 10% palladium on charcoal as catalyst. After 30 minutes the catalyst was filtered off and 0.98 g of (ZX)-2-amino-α-[[2-(diphenylmethoxy)-1,1-dimethyl-2-oxoethoxy]imino]-4-thiazoleacetic acid, 27 mg of N-hydroxybenzotriazole and 0.84 g of dicyclohexylcarbodiimide were added to the mother liquor. Following the procedure of example 36 yielded 0.28 g of the title compound, melting point 228°C, *dec.*

Example 45

[3S(Z)]-2-Amino-N-[1-[[[[[(2-oxo-1-imidazolidinyl)carbonyl]amino]sulfonyl]amino]carbonyl]-2-oxo-3-azetidinyl]-α-(methoxyimino)-4-thiazoleacetamide, potassium salt

A) (S)-[1-[[[[[(2-Oxo-1-imidazolidinyl)carbonyl]amino]sulfonyl]amino]carbonyl]-2-oxo-3-azetidinyl]carbamic acid, phenylmethyl ester, potassium salt

(S)-[1-[[[[[[2-Oxo-3-(triphenylmethyl)-1-imidazolidinyl]carbonyl]amino]sulfonyl]amino]carbonyl]-2-oxo-3-azetidinyl]carbamic acid, phenylmethyl ester, potassium salt (1.7 g; see example 44A) was treated with 2N phosphoric acid and 1N potassium hydroxide following the procedure described in example 34A to yield 1.73 g of the title compound.

B) [3S(Z)]-2-Amino-N-[1-[[[[[(2-oxo-1-imidazolidinyl)carbonyl]amino]sulfonyl]amino]carbonyl]-2-oxo-3-azetidinyl]-α-(methoxyimino)-4-thiazoleacetamide, potassium salt

(S)-[1-[[[[[(2-Oxo-1-imidazolidinyl)carbonyl]amino]sulfonyl]amino]carbonyl]-2-oxo-3-azetidinyl]-carbamic acid, phenylmethyl ester, potassium salt (0.8 g) was hydrogenated in 70 ml of dry dimethylformamide using 0.5 g of 10% palladium on charcoal as catalyst. After 20 minutes the catalyst was filtered off, and (Z)-2-amino-α-(methoxyimino)-4-thiazoleacetic acid (0.38 g), 27 mg of N-hydroxybenzotriazole and 0.78 g of dicyclohexylcarbodiimide were added to the mother liquor. The solution was stirred for 14 hours at room temperature and the solvent was then removed *in vacuo*. The residue was chromatographed on HP-20 resin, to yield 200 mg of the title compound, melting point 216°C, *dec.*

Example 46

[3S(Z)]-2-Amino-N-[1-[[[[[3-(methylsulfonyl)-2-oxo-1-imidazolidinyl]carbonyl]-amino]sulfonyl]amino]carbonyl]-2-oxo-3-azetidinyl-α-(methoxyimino)-4-thiazoleacetamide, potassium salt

A) (S)-[1-[[[[[[3-(Methylsulfonyl-2-oxo-1-imidazolidinyl]carbonyl]amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (3.0 g) was suspended in 300 ml of dry tetrahydrofuran and cooled to −70°C. Chlorosulfonylisocyanate (2.1 g) dissolved in 10 ml of dry tetrahydrofuran was dropped in with stirring. The temperature was allowed to rise to 0°C and this temperature was maintained for 10 minutes, then cooled to −40°C. Triethylamine (1.7 g) and 2.3 g of 1-carbamoyl-3-(methylsulfonyl)-2-oxoimidazolidine were added. Following the procedure described in example 34A yielded 2.8 g of the title compound.

B) [3S(Z)]-2-Amino-N-[1-[[[[[3-(methylsulfonyl)-2-oxo-1-imidazolidinyl]carbonyl]amino]sulfonyl]amino]carbonyl-2-oxo-3-azetidinyl]-α-(methoxyimino)-4-thiazoleacetamide, potassium salt potassium salt

(S)-[1-[[[[[[3-(Methylsulfonyl)-2-oxo-1-imidazolidinyl]carbonyl]amino]sulfonyl]amino]carbonyl]-2-oxo-3-azetidinyl]carbamic acid, phenylmethyl ester, potassium salt (1.8 g) was hydrogenated in 300 ml of dry dimethylformamide using 2.0 g of 10% palladium on charcoal as catalyst. After 20 minutes the catalyst was filtered off, and (Z)-2-amino-α-(methoxyimino)-4-thiazoleacetic acid (1.1 g), 70 mg of N-hydroxybenzotriazole and 2.0 g of dicyclohexylcarbodiimide were added to the mother liquor. The solution was stirred for 12 hours at room temperature and the solvent was then removed *in vacuo*. The residue was chromatographed on HP-20 resin, eluting with water/acetone (9:1), to yield 200 mg of ther title compound, melting point 204°C, *dec.*

Example 47

[3S(Z)]-2-[[[2-[[1-[[[[[[3-(Methylsulfonyl)-2-oxo-1-imidazolidinyl]carbonyl]amino]sulfonyl]amino]carbonyl]-2-oxo-3-azetidinyl]amino-1-(2-amino-4-thiazolyl)-2-oxoethylidene]amino]oxy]-2-methylpropanoic acid, dipotassium salt

(S)-[1-[[[[[[3-(Methylsulfonyl)-2-oxo-1-imidazolidinyl]carbonyl]amino]sulfonyl]amino]carbonyl]-2-oxo-3-azetidinyl]carbamic acid, phenylmethyl ester, potassium salt (1.5 g; see example 46A) was hydrogenated in 150 ml of dry dimethylformamide using 0.9 g of 10% palladium on charcoal as catalyst. After 20 minutes the catalyst was filtered off and 1.23 g of (Z)-2-amino-α-[[2-(diphenylmethoxy)-1,1-dimethyl-2-oxoethoxy]-imino)-4-thiazoleacetic acid, 40 mg of N-hydroxybenzotriazole and 1.1 g of dicyclohexylcarbodiimide were added to the mother liquor. Following the procedure of example 36 yielded 180 mg of the title compound, melting point 236°C, *dec.*

Example 48

[3S(Z)]-2-Amino-N-[1-[[[[[(3-isopropyl-2-oxo-1-imidazolidinyl)carbonyl]amino]sulfonyl]amino]carbonyl]-amino]sulfonyl]amino]carbonyl]-2-oxo-3-azetidinyl]-α-(methoxyimino)-4-thiazoleacetamide, potassium salt

A) (S)-[1-[[[[[(3-Isopropyl-2-oxo-1-imidazolidinyl)carbonyl]amino]sulfonyl]amino]carbonyl]-2-oxo-3-azetidinyl]carbamic acid, phenylmethyl ester, potassium salt

(S)-3-[[(Phenylmethoxy)carbonyl]amino]-2-azetidinone (3.0 g) was suspended in 300 ml of dry

tetrahydrofuran and cooled to −70°C. Chlorosulfonylisocyanate (2.1 g) dissolved in 10 ml of dry tetrahydrofuran was dropped in with stirring. The temperature was allowed to rise to 0°C and this temperature was maintained for 10 minutes, then cooled to −40°C. Triethylamine (1.7 g) and 2.74 g of 1-carbamoyl-3-isopropyl-2-oxo-imidazolidine were added. Following the procedure described in example 34A yielded 5.9 g of the title compound.

B) [3S(Z)]-2-Amino-N-[1-[[[[(3-isopropyl-2-oxo-1-imidazolidinyl)carbonyl]amino]sulfonyl]amino]carbonyl]-2-oxo-3-azetidinyl]-α-(methoxyimino)-4-thiazoleacetamide, potassium salt

(S)-[1-[[[[(3-Isopropyl-2-oxo-1-imidazolidinyl)carbonyl]amino]sulfonyl]amino]carbonyl]-2-oxo-3-azetidinyl]carbamic acid, phenylmethyl ester, potassium salt (1.5 g) was hydrogenated in 150 ml of dry dimethylformamide using 0.8 g of 10% palladium on charcoal as catalyst. After 20 minutes the catalyst was filtered, off, and 0.6 g of (Z)-2-amino-α-(methoxyimino)-4-thiazoleacetic acid, 40 mg of N-hydroxybenzotriazole and 1.2 g of dicyclohexylcarbodiimide were added to the mother liquor. The solution was stirred for 14 hours at room temperature and the solvent was then removed *in vacuo*. The residue was chromatographed on HP-20 resin, eluting with water/acetone (9.25:0.75) to yield 300 mg of the title compound, melting point 189°C, *dec.*

Example 49

[3S(Z)]-2-[[[2-[[1-[[[[(3-isopropyl-2-oxo-1-imidazolidinyl)carbonyl]amino]sulfonyl]amino]carbonyl-2-oxo-3-azetidinyl]amino]-1-(2-amino-4-thiazolyl)-2-oxoethylidene]amino]oxy]-2-methylpropanoic acid, dipotassium salt

(S)-[1-[[[[(3-Methyl-2-oxo-1-imidazolidinyl)carbonyl]amino]sulfonyl]amino]carbonyl]-2-oxo-3-azetidinyl]carbamic acid, phenylmethyl ester, potassium salt (1.5 g; see example 42A) was hydrogenated in 150 ml of dry dimethylformamide using 0.8 g of 10% palladium on charcoal as catalyst. After 20 minutes the catalyst was filtered off and 1.3 g of (Z)-2-amino-α-[[2-(diphenylmethoxy)-1,1-dimethyl-2-oxoethoxy]imino]-4-thiazoleacetic acid, 40 mg of N-hydroxybenzotriazole and 1.2 g of dicyclohexylcarbodiimide were added to the mother liquor. Following the procedure of example 36 yielded 0.4 g of the title compound, melting point 234°C, *dec.*.

**Claims for the Contracting States: BE CH DE FR GB IT LI LU NL SE**

1. A compound having the formula

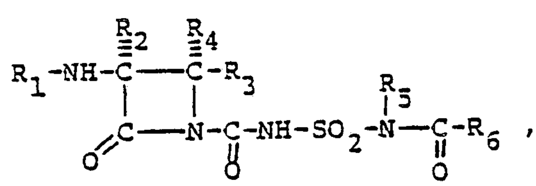

or a salt thereof, wherein

$R_1$ is an acyl group commonly employed in betalactam antibiotics;

$R_2$ is hydrogen or methoxy;

$R_3$ and $R_4$ are the same or different and each is hydrogen, alkyl or aryl;

$R_5$ is hydrogen, alkyl or aryl;

$R_6$ is hydrogen, alkyl, aryl, a 5, 6 or 7-membered heterocycle, $-NR_7R_8$, or $-(CH_2)_n-X$ wherein n is 1, 2, 3 or 4 and X is halogen, aryl, alkoxy, aryloxy or $-NR_9R_{10}$;

$R_7$ and $R_8$ are the same or different and each is hydrogen, alkyl or aryl, or $R_7$ is hydrogen and $R_8$ is a 5, 6 or 7-membered heterocycle or $-(CH_2)_n-Y$ wherein n is 1, 2, 3 or 4 and Y is alkoxy, amino, alkylthio or halogen; and

$R_9$ and $R_{10}$ are the same or different and each is hydrogen or alkyl, or $R_9$ is hydrogen and $R_{10}$ is a 5, 6 or 7-membered heterocycle, and wherein the above definitions are as follows:

alkyl and alkoxy refers to groups having 1 to 10 carbon atoms,

aryl refers to phenyl and phenyl substituted with 1, 2 or 3 amino($-NH_2$), halogen, hydroxyl, trifluoromethyl, $C_1-C_4$ alkyl, $C_1-C_4$ alkoxy or carbonyl, and a 5, 6 or 7-membered heterocycle refers to aromatic and non-aromatic groups containing one or more nitrogen, oxygen or sulfur atoms, and, provided $R_6$ is $NR_7R_8$ or a heterocycle, optionally substituted by oxo(=O), halogen, hydroxy, nitro, amino, cyano, trifluoromethyl, $C_1-C_4$ alkyl, $C_1-C_4$ alkoxy, alkylsulfonyl, phenyl, phenyl substituted with 1, 2 or 3 groups selected from amino($-NH_2$), halogen, hydroxyl, trifluoromethyl, $C_1-C_4$ alkyl, $C_1-C_4$ alkoxy and carboxyl, 2-furylmethyleneimino

( 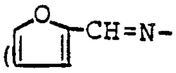 ),

phenylmethyleneimino and $C_1-C_4$ alkyl substituents, the latter being substituted with one, or more, azido, amino ($-NH_2$), halogen, hydroxy, carboxy, cyano, alkoxycarbonyl, aminocarbonyl, alkanoyloxy having 2 to

10 carbon atoms, alkoxy, aryloxy, unsubstituted 5, 6 or 7-membered heterocycleoxy, mercapto, alkylthio, arylthio, alkylsulfinyl, or alkylsulfonyl groups, wherein the aryl, alkoxy and alkyl moieties are as defined above.

2. A compound having the formula

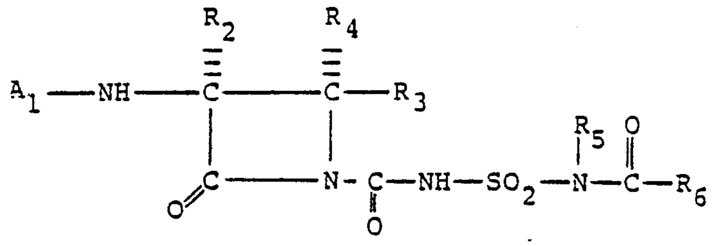

wherein $R_2$, $R_3$, $R_4$, $R_5$ and $R_6$ are defined in claim 1 and $A_1$—NH— is a protected amino group or $A_1$— is hydrogen.

3. A compound in accordance with claims 1 and 2 wherein $R_2$ is hydrogen.

4. A compound in accordance with claims 1 and 2 wherein $R_5$ is hydrogen.

5. A compound in accordance with claims 1 and 2 wherein $R_3$ and $R_4$ are each hydrogen.

6. A compound in accordance with claims 1 and 2 wherein $R_2$, $R_3$, $R_4$ and $R_5$ are each hydrogen.

7. A compound in accordance with claim 6 wherein $R_6$ is hydrogen.

8. A compound in accordance with claim 6 wherein $R_6$ is $C_1$—$C_{10}$ alkyl.

9. A compound in accordance with claim 6 wherein $R_6$ is aryl as defined in claim 1.

10. A compound in accordance with claim 6 wherein $R_6$ is a 5, 6 or 7-membered heterocycle.

11. A compound in accordance with claim 6 wherein $R_6$ is 4-$C_1$—$C_{10}$-alkyl-2,3-dioxo-1-piperazinyl.

12. A compound in accordance with claim 6 wherein $R_6$ is 2-oxo-1-imidazolidinyl.

13. A compound in accordance with claim 6 wherein $R_6$ is 3-$C_1$—$C_{10}$-alkyl-2-oxo-1-imidazolidinyl.

14. A compound in accordance with claim 6 wherein $R_6$ is 3-(substituted $C_1$—$C_{10}$-alkyl)-2-oxo-1-imidazolidinyl.

15. A compound in accordance with claim 14 wherein $R_6$ is 3-(2-aminoethyl)-2-oxo-1-imidazolidinyl.

16. A compound in accordance with claim 6 wherein $R_1$ is (Z)-2-amino-α-($C_1$—$C_{10}$-alkoxyimino)-4-thiazoleacetyl.

17. A compound in accordance with claim 16 wherein $R_1$ is (Z)-2-amino-α-(methoxyimino)-4-thiazole-acetyl.

18. A compound in accordance with claim 16 wherein $R_1$ is (Z)-2-amino-α-[(1-carboxy-1-methylethoxy)-imino]-4-thiazoleacetyl.

19. [3S(Z)]-2-[[[1-(2-amino-4-thiazolyl)-2-[[1-[[[[(3-ethyl-2-oxo-1-imidazolidinyl)carbonyl]amino]-sulfonyl]amino]carbonyl]-2-oxo-3-azetidinyl]amino]-2-oxoethylidene]amino]oxy]-2-methylpropanoic acid, or a salt thereof.

20. [3S(Z)]-2-[[[1-(2-amino-4-thiazolyl)-2-[[1-[[[[(3-ethyl-2-oxo-1-imidazolidinyl)carbonyl]amino]-sulfonyl]amino]carbonyl]-2-oxo-3-azetidinyl]amino]-2-oxoethylidene]amino]oxy]-2-methylpropanoic acid, dipotassium salt.

21. [3S(Z)]-2-[[[2-[[1-[[[[[3-(2-aminoethyl)-2-oxo-1-imidazolidinyl]carbonyl]amino]sulfonyl]amino]-carbonyl]-2-oxo-3-azetidinyl]amino]-1-(2-amino-4-thiazolyl)-2-oxoethylidene]amino]oxy]-2-methyl-propanoic acid, or a salt thereof.

22. [3S(Z)]-2-[[[2-[[1-[[[[[3-[2-aminoethyl)-2-oxo-1-imidazolidinyl]carbonyl]amino]sulfonyl]amino]-carbonyl]-2-oxo-3-azetidinyl]amino]-1-(2-amino-4-thiazolyl)-2-oxoethylidene]amino]oxy]-2-methyl-propanoic acid, dipotassium salt.

23. [3S(Z)]-2-[[[2-[[1-[[[[(3-methyl-2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, or a salt thereof.

24. [3S(Z)]-2-[[[2-[[1-[[[[(3-methyl-2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt.

25. [3S(Z)]-2-[[[2-[[1-[[[[(2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, or a salt thereof.

26. [3S(Z)]-2-[[[2-[[1-[[[[(2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt.

27. A compound in accordance with claims 1 to 26 for use in treating bacterial infections.

28. Pharmaceutical compositions comprising at least one compound in accordance with claim 1 and usual carriers, additives and adjuvants.

29. A process for preparing a compound having the formula

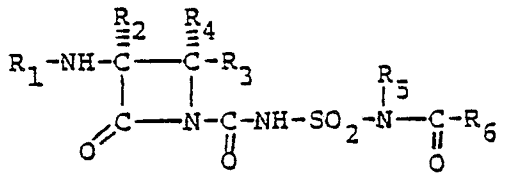

or a salt thereof, wherein $R_1$, $R_2$, $R_3$, $R_4$, $R_5$ and $R_6$ are as defined in claim 1, which comprises reacting a β-lactam having the formula

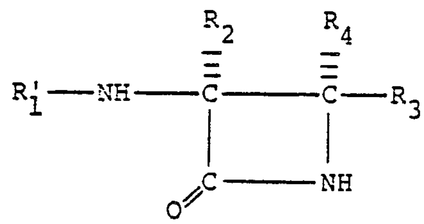

wherein $R_2$, $R_3$ and $R_4$ are as previously defined and $R_1'$ is acyl ($R_1$) as previously defined or the group $R_1'$—NH is a protected amino group with an isocyanate having the formula

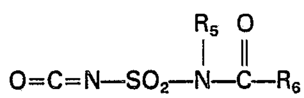

or sequential segments thereof wherein $R_5$ and $R_6$ are as previously defined, and where $R_1'$—NH— is a protected amino group removing said protecting group and acylating with the appropriate acylating group to form the desired product.

30. A process for preparing a compound of the formula

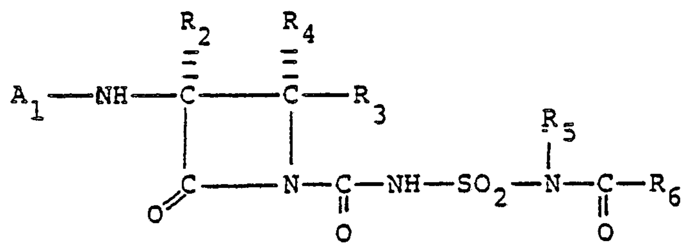

wherein $R_2$, $R_3$, $R_4$, $R_5$ and $R_6$ are as defined in claim 1 and $A_1$—NH— is a protected amino or $A_1$— is hydrogen characterized by reacting a compound of the formula

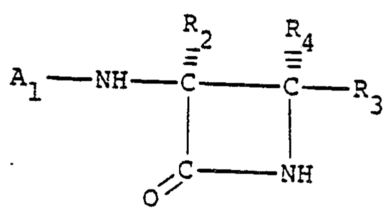

wherein the group $A_1$—NH— is a protected amino group with an isocyanate having the formula

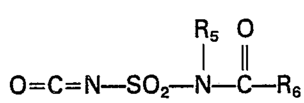

.

or sequential segments thereof to form a product wherein $A_1$—NH— is a protected amino group and removing said protecting group to form a product wherein $A_1$— is hydrogen.

**Claims for the Contracting State: AT**

1. A process for preparing a compound having the formula

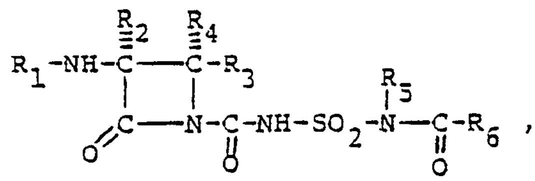

or a salt thereof, wherein

$R_1$ is an acyl group commonly employed in betalactam antibiotics;

$R_2$ is hydrogen or methoxy;

$R_3$ and $R_4$ are the same or different and each is hydrogen, alkyl or aryl;

$R_5$ is hydrogen, alkyl or aryl;

$R_6$ is hydrogen, alkyl, aryl, a 5, 6 or 7-membered heterocycle, $-NR_7R_8$, or $-(CH_2)_n-X$ wherein n is 1, 2, 3 or 4 and X is halogen, aryl, alkoxy, aryloxy or $-NR_9R_{10}$;

$R_7$ and $R_8$ are the same or different and each is hydrogen, alkyl or aryl, or $R_7$ is hydrogen and $R_8$ is a 5, 6 or 7-membered heterocycle or $-(CH_2)_n-Y$ wherein n is 1, 2, 3 or 4 and Y is alkoxy, amino, alkylthio or halogen; and

$R_9$ and $R_{10}$ are the same or different and each is hydrogen or alkyl, or $R_9$ is hydrogen and $R_{10}$ is a 5, 6 or 7-membered heterocycle, and wherein the above definitions are as follows:

alkyl and alkoxy refers to groups having 1 to 10 carbon atoms,

aryl refers to phenyl and phenyl substituted with 1, 2 or 3 amino($-NH_2$), halogen, hydroxyl, trifluoromethyl, $C_1-C_4$ alkyl, $C_1-C_4$ alkoxy or carbonyl, and a 5, 6 or 7-membered heterocycle refers to aromatic and non-aromatic groups containing one or more nitrogen, oxygen or sulfur atoms, and, provided $R_6$ is $NR_7R_8$ or a heterocycle, optionally substituted by oxo(=O), halogen, hydroxy, nitro, amino, cyano, trifluoromethyl, $C_1-C_4$ alkyl, $C_1-C_4$ alkoxy, alkylsulfonyl, phenyl, phenyl substituted with 1, 2 or 3 groups selected from amino($-NH_2$), halogen, hydroxyl, trifluoromethyl, $C_1-C_4$ alkyl, $C_1-C_4$ alkoxy and carboxyl, 2-furylmethyleneimino

( 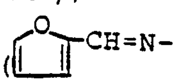 ),

phenylmethyleneimino and $C_1-C_4$ alkyl substituents, the latter being substituted with one, or more, azido, amino ($-NH_2$), halogen, hydroxy, carboxy, cyano, alkoxycarbonyl, aminocarbonyl, alkanoyloxy having 2 to 10 carbon atoms, alkoxy, aryloxy, unsubstituted 5, 6 or 7-membered heterocycleoxy, mercapto, alkylthio, arylthio, alkylsulfinyl, or alkylsulfonyl groups, wherein the aryl, alkoxy and alkyl moieties are as defined above;

which comprises reacting a β-lactam having the formula

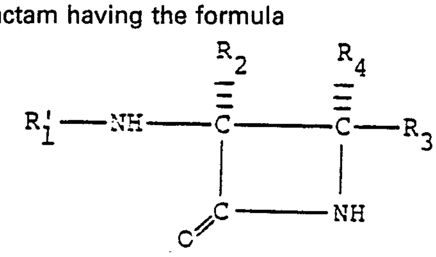

wherein $R_2$, $R_3$ and $R_4$ are as previously defined and $R_1'$ is acyl ($R_1$) as previously defined or the group $R_1'$—NH is a protected amino group with an isocyanate having the formula

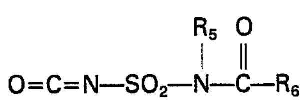

or sequential segments thereof wherein $R_5$ and $R_6$ are as previously defined, and where $R_1'$—NH— is a protected amino group removing said protecting group and acylating with the appropriate acylating group to form the desired product.

2. A process for preparing a compound of the formula

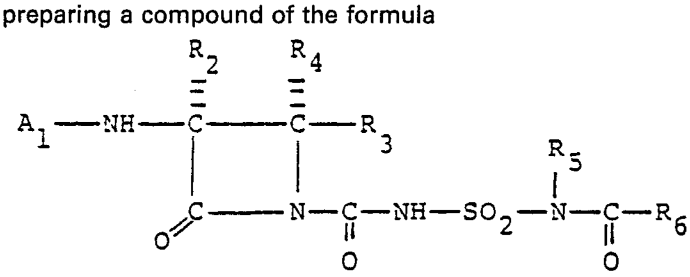

wherein $R_2$, $R_3$, $R_4$, $R_5$ and $R_6$ are as defined in claim 1 and $A_1$—NH— is a protected amino or $A_1$— is hydrogen characterized by reacting a compound of the formula

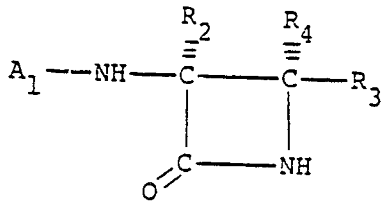

wherein the group $A_1$—NH— is a protected amino group with an isocyanate having the formula

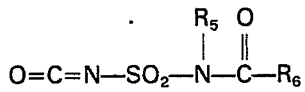

or sequential segments thereof to form a product wherein $A_1$—NH— is a protected amino group and removing said protecting group to form a product wherein $A_1$— is hydrogen.

3. A process for preparing a compound in accordance with claims 1 and 2 wherein $R_2$ is hydrogen.

4. A process for preparing a compound in accordance with claims 1 and 2 wherein $R_5$ is hydrogen.

5. A process for preparing a compound in accordance with claims 1 and 2 wherein $R_3$ and $R_4$ are each hydrogen.

6. A process for preparing a compound in accordance with claims 1 and 2 wherein $R_2$, $R_3$, $R_4$ and $R_5$ are each hydrogen.

7. A process for preparing a compound in accordance with claim 6 wherein $R_6$ is hydrogen.

8. A process for preparing a compound in accordance with claim 6 wherein $R_6$ is $C_1$—$C_{10}$ alkyl.

9. A process for preparing a compound in accordance with claim 6 wherein $R_6$ is aryl as defined in claim 1.

10. A process for preparing a compound in accordance with claim 6 wherein $R_6$ is a 5, 6 or 7-membered heterocycle.

11. A process for preparing a compound in accordance with claim 6 wherein $R_6$ is 4-$C_1$—$C_{10}$-alkyl-2,3-dioxo-1-piperazinyl.

12. A process for preparing a compound in accordance with claim 6 wherein $R_6$ is 2-oxo-1-imidazolidinyl.

13. A process for preparing a compound in accordance with claim 6 wherein $R_6$ is 3-$C_1$—$C_{10}$-alkyl-2-oxo-1-imidazolidinyl.

14. A process for preparing a compound in accordance with claim 6 wherein $R_6$ is 3-(substituted $C_1$—$C_{10}$-alkyl)-2-oxo-1-imidazolidinyl.

15. A process for preparing a compound in accordance with claim 14 wherein $R_6$ is 3-(2-aminoethyl)-2-oxo-1-imidazolidinyl.

16. A process for preparing a compound in accordance with claim 6 wherein $R_1$ is (Z)-2-amino-α-($C_1$—$C_{10}$-alkoxyimino)-4-thiazoleacetyl.

17. A process for preparing a compound in accordance with claim 16 wherein $R_1$ is (Z)-2-amino-α-(methoxyimino)-4-thiazoleacetyl.

18. A process for preparing a compound in accordance with claim 16 wherein $R_1$ is (Z)-2-amino-α-[(1-carboxy-1-methylethoxy)imino]-4-thiazoleacetyl.

19. A process in accordance with claims 1 and 2 for preparing [3S(Z)]-2-[[[1-(2-amino-4-thiazolyl)-2-[[1-[[[[(3-ethyl-2-oxo-1-imidazolidinyl)carbonyl]amino]sulfonyl]amino]carbonyl]-2-oxo-3-azetidinyl]amino]-2-oxoethylidene]amino]oxy]-2-methylpropanoic acid, or a salt thereof.

20. A process in accordance with claims 1 and 2 for preparing [3S(Z)]-2-[[[1-(2-amino-4-thiazolyl)-2-[[1-[[[[(3-ethyl-2-oxo-1-imidazolidinyl)carbonyl]amino]sulfonyl]amino]carbonyl]-2-oxo-3-azetidinyl]amino]-2-oxoethylidene]amino]oxy]-2-methylpropanoic acid, dipotassium salt.

21. A process in accordance with claims 1 and 2 for preparing [3S(Z)]-2-[[[2-[[1-[[[[[3-(2-aminoethyl)-2-oxo-1-imidazolidinyl]carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, or a salt thereof.

22. A process in accordance with claims 1 and 2 for preparing [3S(Z)]-2-[[[2-[[1-[[[[[3-[2-aminoethyl)-2-oxo-1-imidazolidinyl]carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt.

23. A process in accordance with claims 1 and 2 for preparing [3S(Z)]-2-[[[2-[[1-[[[[(3-methyl-2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, or a salt thereof.

24. A process in accordance with claims 1 and 2 for preparing [3S(Z)]-2-[[[2-[[1-[[[[(3-methyl-2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt.

25. A process in accordance with claims 1 and 2 for preparing [3S(Z)]-2-[[[2-[[1-[[[[(2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, or a salt thereof.

26. A process in accordance with claims 1 and 2 for preparing [3S(Z)]-2-[[[2-[[1-[[[[(2-oxo-1-imidazolidinyl)carbonyl]amino]sulfonyl]amino]carbonyl]-2-oxo-3-azetidinyl]amino]-1-(2-amino-4-thiazolyl)-2-oxoethylidene]amino]oxy]-2-methylpropanoic acid, dipotassium salt.

**Patentansprüche für die Vertragsstaaten: BE CH DE FR GB IT LI LU NL SE**

1. Verbindung der Formel

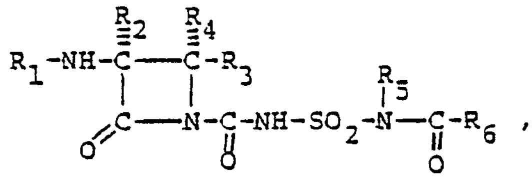

oder ein Salz davon, in der

$R_1$ ein üblicher in Betalactam-Antibiotika verwendeter Acylrest ist;

$R_2$ ein Wasserstoffatom oder eine Methoxygruppe ist;

$R_3$ und $R_4$ gleich oder verschieden sind und jeweils ein Wasserstoffatom, Alkyl oder Aryl bedeuten,

$R_5$ ein Wasserstoffatom, Alkyl oder Aryl ist;

$R_6$ ein Wasserstoffatom, Alkyl, Aryl, ein Heterocyclus mit 5, 6 oder 7 Ringatomen, die Gruppe $-NR_7R_8$ oder $-(CH_2)_n-X$ ist, wobei n den Wert 1, 2, 3 oder 4 hat und X Halogen, Aryl, Alkoxy, Aryloxy oder $-NR_9R_{10}$ bedeutet;

$R_7$ und $R_8$ gleich oder verschieden sind und jeweils ein Wasserstoffatom, Alkyl oder Aryl bedeuten, oder $R_7$ ein Wasserstoffatom ist und $R_8$ ein Heterocyclus mit 5, 6 oder 7 Ringatomen oder die Gruppe $-(CH_2)_n-Y$ ist, wobei n den Wert 1, 2, 3 oder 4 hat, und Y Alkoxy, Amino, Alkylthio oder Halogen bedeutet; und

$R_9$ und $R_{10}$ gleich oder verschieden sind und jeweils ein Wasserstoffatom oder Alkyl bedeuten oder $R_9$ ein Wasserstoffatom und $R_{10}$ ein Heterocyclus mit 5, 6 oder 7 Ringatomen ist, und die vorstehend angegebenen Definitionen folgende Bedeutung haben:

"Alkyl" und "Alkoxy" bedeuten Gruppen mit 1 bis 10 Kohlenstoffatomen,

"Aryl" bedeutet Phenyl und Phenyl substituiert mit 1, 2 oder 3 der Gruppen Amino($-NH_2$), Halogen, Hydroxyl, Trifluormethyl, $C_1-C_4$-Alkyl, $C_1-C_4$-Alkoxy oder Carboxyl, und "ein Heterocyclus mit 5, 6 oder 7 Ringatomen" bedeutet aromatische und nicht-aromatische Gruppen, die ein oder mehrere Stickstoff-, Sauerstoff- oder Schwefelatome enthalten, und, mit der Maßgabe, daß $R_6$ eine Gruppe $NR_7R_8$ oder ein Heterocyclus ist, gegebenenfalls substituiert sind durch Oxo(=O)-, Halogen-, Hydroxy-, Nitro-, Amino-, Cyano-, Trifluormethyl-, $C_1-C_4$-Alkyl, $C_1-C_4$-Alkoxy-, Alkylsulfonyl-, Phenyl-, Phenyl substituiert mit 1, 2 oder 3 der Gruppen Amino($-NH_2$), Halogen, Hydroxyl, Trifluormethyl, $C_1-C_4$-Alkyl, $C_1-C_4$-Alkoxy oder Carboxyl, 2-Furylmethylenimino

( 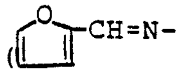 ),

Phenylmethylenimino und $C_1-C_4$-Alkyl-Substituenten, letztere substituiert mit einer oder mehreren Azido-, Amino($-NH_2$)-, Halogen, Hydroxy-, Carboxy-, Cyano-, Alkoxycarbonyl-, Aminocarbonyl-, Alkanoyloxy mit 2 bis 10 Kohlenstoffatomen, Alkoxy-, Aryloxy, nicht substituierten Heterocyclooxy mit 5, 6 oder 7 Ringatomen, Mercapto, Alkylthio-, Arylthio-, Alkylsulfinyl- oder Alkylsulfonylgruppen, in denen die Aryl-, Alkoxy- und Alkylreste die vorstehend angegebene Bedeutung haben.

2. Verbindung der Formel

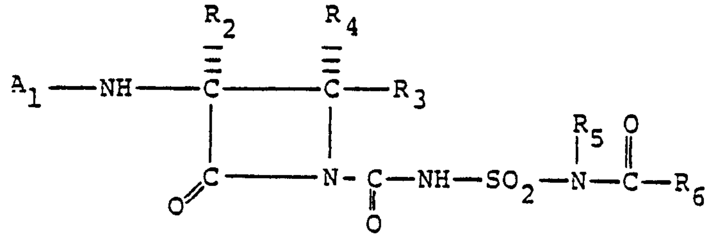

in der $R_2$, $R_3$, $R_4$, $R_5$ und $R_6$ die in Anspruch 1 angegebene Bedeutung haben und $A_1$—NH— eine geschützte Aminogruppe ist oder $A_1$— ein Wasserstoffatom bedeutet.

3. Verbindung nach Anspruch 1 und 2, in der $R_2$ ein Wasserstoffatom ist.

4. Verbindung nach Anspruch 1 und 2, in der $R_5$ ein Wasserstoffatom ist.

5. Verbindung nach Anspruch 1 und 2, in der $R_3$ und $R_4$ jeweils ein Wasserstoffatom sind.

6. Verbindung nach Anspruch 1 und 2, in der $R_2$, $R_3$, $R_4$ und $R_5$ jeweils ein Wasserstoffatom sind.

7. Verbindung nach Anspruch 6, in der $R_6$ ein Wasserstoffatom ist.

8. Verbindung nach Anspruch 6, in der $R_6$ ein $C_1$—$C_{10}$-Alkylrest ist.

9. Verbindung nach Anspruch 6, in der $R_6$ ein wie bei Anspruch 1 angegebener Arylrest ist.

10. Verbindung nach Anspruch 6, in der $R_6$ ein Heterocyclus mit 5, 6 oder 7 Ringatomen ist.

11. Verbindung nach Anspruch 6, in der $R_6$ ein 4-$C_1$—$C_{10}$-Alkyl-2,3-dioxo-1-piperazinylrest ist.

12. Verbindung nach Anspruch 6, in der $R_6$ eine 2-Oxo-1-imidazolidinylgruppe ist.

13. Verbindung nach Anspruch 6, in der $R_6$ ein 3-$C_1$—$C_{10}$-Alkyl-2-oxo-1-imidazolidinylrest ist.

14. Verbindung nach Anspruch 6, in der $R_6$ ein 3-(substituiertes $C_1$—$C_{10}$-Alkyl)-2-oxo-1-imidazolidinylrest ist.

15. Verbindung nach Anspruch 14, in der $R_6$ ein 3-(2-Aminoäthyl)-2-oxo-1-imidazolidinylrest ist.

16. Verbindung nach Anspruch 6, in der $R_1$ ein (Z)-2-Amino-α-($C_1$—$C_{10}$-alkoxyimino)-4-thiazolacetylrest ist.

17. Verbindung nach Anspruch 16, in der $R_1$ ein (Z)-2-Amino-α-(methoxyimino)-4-thiazolacetylrest ist.

18. Verbindung nach Anspruch 16, in der $R_1$ ein (Z)-2-Amino-α-[(1-carboxy-1-methyläthoxy)imino]-4-thiazolacetylrest ist.

19. [3S(Z)]-2-[[[1-(2-Amino-4-thiazolyl)-2-[[1-[[[[(3-äthyl-2-oxo-1-imidazolidinyl)carbonyl]-amino]-sulfonyl]amino]carbonyl]-2-oxo-3-azetidinyl])amino]-2-oxoäthyliden]amino]oxy]-2-methylpropionsäure oder ein Salz davon.

20. [3S(Z)]-2-[[[1-(2-Amino-4-thiazolyl)-2-[[1-[[[[(3-äthyl-2-oxo-1-imidazolidinyl)carbonyl]-amino]-sulfonyl]amino]carbonyl]-2-oxo-3-azetidinyl])amino]-2-oxoäthyliden]amino]oxy]-2-methylpropionsäure-dikaliumsalz.

21. [3S(Z)]-2-[[[2-[[1-[[[[[3-(2-Aminoäthyl)-2-oxo-1-imidazolidinyl]-carbonyl]-amino]sulfonyl]-amino]-carbonyl)-2-oxo-3-azetidinyl]amino]-1-(2-amino-4-thiazolyl)-2-oxoäthyliden]amino]oxy]-2-methylpropionsäure oder ein Salz davon.

22. [3S(Z)]-2-[[[2-[[1-[[[[[3-(2-Aminoäthyl)-2-oxo-1-imidazolidinyl]-carbonyl]amino]sulfonyl]-amino]-carbonyl)-2-oxo-3-azetidinyl]amino]-1-(2-amino-4-thiazolyl)-2-oxoäthyliden]amino]oxy]-2-methylpropionsäure-dikaliumsalz.

23. [3S(Z)]-2-[[[2-[[1-[[[[(3-Methyl-2-oxo-1-imidazolidinyl)carbonyl]amino]sulfonyl]amino]-carbonyl]-2-oxo-3-azetidinyl]amino]-1-(2-amino-4-thiazolyl)-2-oxoäthyliden]amino]oxy]-2-methylpropionsäure oder ein Salz davon.

24. [3S(Z)]-2-[[[2-[[1-[[[[(3-Methyl-2-oxo-1-imidazolidinyl)carbonyl]amino]sulfonyl]amino]-carbonyl]-2-oxo-3-azetidinyl]amino]-1-(2-amino-4-thiazolyl)-2-oxoäthyliden]amino]oxy]-2-methyl-propionsäure-dikaliumsalz.

25. [3S(Z)]-2-[[[2-[[1-[[[[(2-Oxo-1-imidazolidinyl)-carbonyl]amino]sulfonyl]amino]carbonyl]-2-oxo-3-azetidinyl]amino]-1-(2-amino-4-thiazolyl)-2-oxoäthyliden]amino]oxy]-2-methylpropionsäure oder ein Salz davon.

26. [3S(Z)]-2-[[[2-[[1-[[[[(2-Oxo-1-imidazolidinyl)-carbonyl]amino]sulfonyl]amino]carbonyl]-2-oxo-3-azetidinyl]amino]-1-(2-amino-4-thiazolyl)-2-oxoäthyliden]amino]oxy]-2-methylpropionsäure-dikaliumsalz.

27. Verbindung nach Anspruch 1 bis 26 zur Verwendung bei der Behandlung bakterieller Infektionen.

28. Arzneimittel, enthaltend mindestens eine Verbindung nach Anspruch 1 und übliche Träger, Zusatzstoffe und Hilfsstoffe.

29. Verfahren zur Herstellung einer Verbindung der Formel

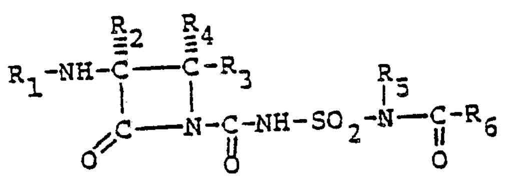
,

oder eines Salzes davon, in der $R_1$, $R_2$, $R_3$, $R_4$, $R_5$ und $R_6$ die in Anspruch 1 angegebene Bedeutung haben, bei dem man ein β-Lactam der Formel

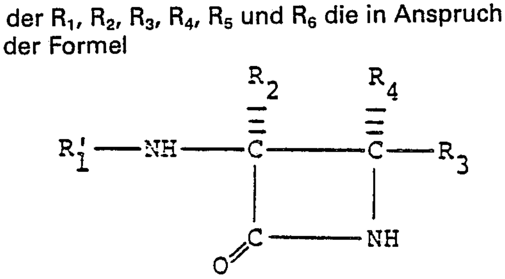

in der $R_2$, $R_3$ und $R_4$ die vorstehend angegebene Bedeutung haben und $R_1'$ ein wie vorstehend angegebener Acylrest ($R_1$) ist oder die Gruppe $R_1'$—NH eine geschützte Aminogruppe ist, mit einem Isocyanat der Formel

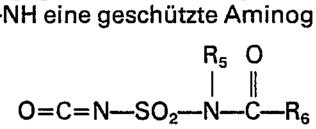

oder mit sequentiellen Segmenten davon umsetzt, wobei $R_5$ und $R_6$ die vorstehend angegebene Bedeutung haben, und, falls $R_1'$—NH— eine geschützte Aminogruppe ist, die Schutzgruppe abspaltet und mit der passenden Acylierungsgruppe acyliert, um das gewünschte Produkt zu bilden.

30. Verfahren zur Herstellung einer Verbindung der Formel

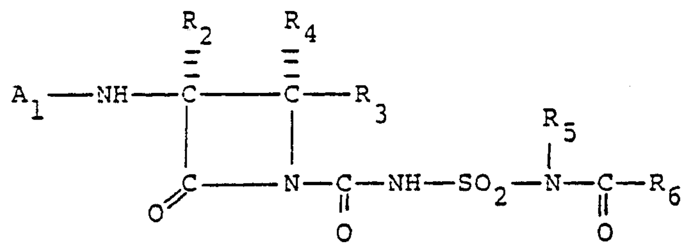

in der $R_2$, $R_3$, $R_4$, $R_5$ und $R_6$ die in Anspruch 1 angegebene Bedeutung haben und $A_1$—NH— eine geschützte Aminogruppe ist oder $A_1$— ein Wasserstoffatom bedeutet, dadurch gekenzeichnet, daß man die Verbindung der Formel

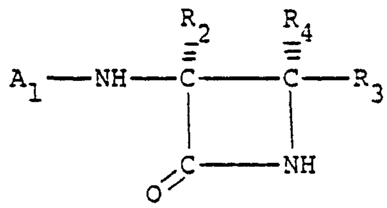

in der die Gruppe $A_1$—NH— eine geschützte Aminogruppe ist, mit einem Isocyanat der Formel

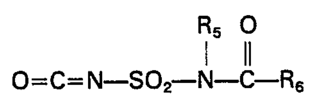

oder mit sequentiellen Segmenten davon umsetzt, um ein Produkt zu bilden, in dem $A_1$—NH— eine geschützte Aminogruppe ist, und die Schutzgruppe abspaltet, um ein Produkt zu bilden, in dem $A_1$— ein Wasserstoffatom ist.

**Patentansprüche für den Vertragsstaat: AT**

1. Verfahren zur Herstellung einer Verbindung der Formel

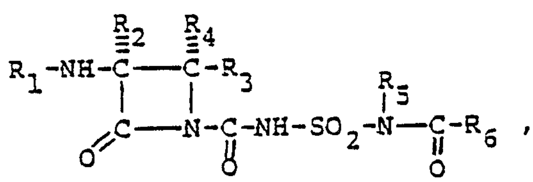

oder eines Salzes davon, in der

$R_1$ ein üblicher in Betalactam-Antibiotika verwendeter Acylrest ist;

$R_2$ ein Wasserstoffatom oder eine Methoxygruppe ist;

$R_3$ und $R_4$ gleich oder verschieden sind und jeweils ein Wasserstoffatom, Alkyl oder Aryl bedeuten,

$R_5$ ein Wasserstoffatom, Alkyl oder Aryl ist;

$R_6$ ein Wasserstoffatom, Alkyl, Aryl, ein Heterocyclus mit 5, 6 oder 7 Ringatomen, die Gruppe $—NR_7R_8$ oder $—(CH_2)_n—X$ ist, wobei n den Wert 1, 2, 3 oder 4 hat und X Halogen, Aryl, Alkoxy, Aryloxy oder $—NR_9R_{10}$ bedeutet;

$R_7$ und $R_8$ gleich oder verschieden sind und jeweils ein Wasserstoffatom, Alkyl oder Aryl bedeuten, oder $R_7$ ein Wasserstoffatom ist und $R_8$ ein Heterocyclus mit 5, 6 oder 7 Ringatomen oder die Gruppe $—(CH_2)_n—Y$ ist, wobei n den Wert 1, 2, 3 oder 4 hat, und Y Alkoxy, Amino, Alkylthio oder Halogen bedeutet; und

$R_9$ und $R_{10}$ gleich oder verschieden sind und jeweils ein Wasserstoffatom oder Alkyl bedeuten oder $R_9$ ein Wasserstoffatom und $R_{10}$ ein Heterocyclus mit 5, 6 oder 7 Ringatomen ist, und die vorstehend angegebenen Definitionen folgende Bedeutungen haben:

"Alkyl" und "Alkoxy" bedeuten Gruppen mit 1 bis 10 Kohlenstoffatomen,

"Aryl" bedeutet Phenyl und Phenyl substituiert mit 1, 2 oder 3 der Gruppen Amino($—NH_2$), Halogen, Hydroxyl, Trifluoromethyl, $C_1—C_4$-Alkyl, $C_1—C_4$-Alkoxy oder Carboxyl, und "ein Heterocyclus mit 5, 6 oder 7 Ringatomen" bedeutet aromatische und nicht-aromatische Gruppen, die ein oder mehrere Stickstoff-, Sauerstoff- oder Schwefelatome enthalten, und, mit der Maßgabe, daß $R_6$ eine Gruppe $NR_7R_8$ oder ein Heterocyclus ist, gegebenenfalls substituiert sind durch Oxo(=O)-, Halogen-, Hydroxy-, Nitro-, Amino-, Cyano-, Trifluormethyl-, $C_1—C_4$-Alkyl, $C_1—C_4$-Alkoxy-, Alkylsulfonyl-, Phenyl-, Phenyl substituiert mit 1, 2

oder 3 der Gruppen Amino(—$NH_2$), Halogen, Hydroxyl, Trifluormethyl, $C_1$—$C_4$-Alkyl, $C_1$—$C_4$-Alkoxy oder Carboxyl, 2-Furylmethylenimino

(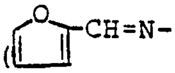),

Phenylmethylenimino und $C_1$—$C_4$-Alkyl-Substituenten, letztere substituiert mit einer oder mehreren Azido-, Amino(—$NH_2$)-, Halogen, Hydroxy-, Carboxy-, Cyano-, Alkoxycarbonyl-, Aminocarbonyl-, Alkanoyloxy mit 2 bis 10 Kohlenstoffatomen, Alkoxy-, Aryloxy, nicht substituierten Heterocyclooxy mit 5, 6 oder 7 Ringatomen, Mercapto, Alkylthio-, Alkylsulfinyl- oder Alkylsulfonylgruppen, in denen die Aryl-, Alkoxy- und Alkylreste die vorstehend angegebene Bedeutung haben, bei dem man ein β-Lactam der Formel

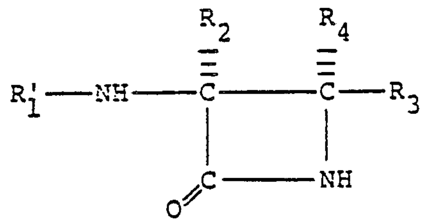

in der $R_2$, $R_3$ und $R_4$ die vorstehen angegebenen Bedeutung haben und $R_1'$ ein wie vorstehend angegebener Acylrest ($R_1$) ist oder die Gruppe $R_1'$—NH eine geschützte Aminogruppe ist, umsetzt mit einem Isocyanat der Formel

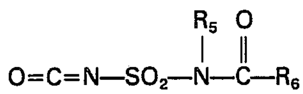

oder mit sequentiellen Segmenten davon, wobei $R_5$ und $R_6$ die vorstehend angegebene Bedeutung haben, und, falls $R_1'$—NH— eine geschützte Aminogruppe ist, die Schutzgruppe abspaltet und mit der passenden Acylierungsgruppe acyliert, um das gewünschte Produkt zu bilden.

2. Verfahren zur Herstellung einer Verbindung der Formel

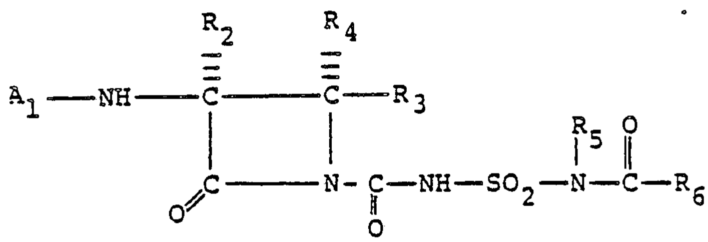

in der $R_2$, $R_3$, $R_4$, $R_5$ und $R_6$ die vorstehend in Anspruch 1 angegebene Bedeutung haben und $A_1$—NH— eine geschützte Aminogruppe ist oder $A_1$— ein Wasserstoffatom bedeutet, dadurch gekenzeichnet, daß man eine Verbindung der Formel

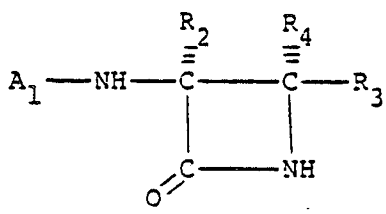

in der die Gruppe $A_1$—NH— eine geschützte Aminogruppe ist, umsetzt mit einem Isocyanat der Formel

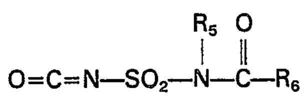

oder mit sequentiellen Segmenten davon, um ein Produkt zu bilden, in dem $A_1$—NH— eine geschützte Aminogruppe ist, und die Schutzgruppe abspaltet, um ein Produkt zu erhalten, in dem $A_1$— ein Wasserstoffatom ist.

3. Verfahren zur Herstellung einer Verbindung nach Anspruch 1 und 2, in der $R_2$ ein Wasserstoffatom ist.

4. Verfahren zur Herstellung einer Verbindung nach Anspruch 1 und 2, in der $R_5$ ein Wasserstoffatom ist.

5. Verfahren zur Herstellung einer Verbindung nach Anspruch 1 und 2, in der $R_3$ und $R_4$ jeweils ein Wasserstoffatom sind.

6. Verfahren zur Herstellung einer Verbindung nach Anspruch 1 und 2, in der $R_2$, $R_3$, $R_4$ und $R_5$ jeweils ein Wasserstoffatom sind.

7. Verfahren zur Herstellung einer Verbindung nach Anspruch 6, in der $R_6$ ein Wasserstoffatom ist.

8. Verfahren zur Herstellung einer Verbindung nach Anspruch 6, in der $R_6$ ein $C_1$—$C_{10}$-Alkylrest ist.

9. Verfahren zur Herstellung einer Verbindung nach Anspruch 6, in der $R_6$ ein wie bei Anspruch 1 angegebener Arylrest ist.

10. Verfahren zur Herstellung einer Verbindung nach Anspruch 6, in der $R_6$ ein Heterocyclus mit 5, 6 oder 7 Ringatomen ist.

11. Verfahren zur Herstellung einer Verbindung nach Anspruch 6, in der $R_6$ ein 4-$C_1$—$C_{10}$-Alkyl-2,3-dioxo-1-piperazinylrest ist.

12. Verfahren zur Herstellung einer Verbindung nach Anspruch 6, in der $R_6$ eine 2-Oxo-1-imidazolidinylgruppe ist.

13. Verfahren zur Herstellung einer Verbindung nach Anspruch 6, in der $R_6$ ein 3-$C_1$—$C_{10}$-Alkyl-2-oxo-1-imidazolidinylrst ist.

14. Verfahren zur Herstellung einer Verbindung nach Anspruch 6, in der $R_6$ ein 3-(substituierter $C_1$—$C_{10}$-Alkylrest)-2-oxo-1-imidazolidinylrest ist.

15. Verfahren zur Herstellung einer Verbindung nach Anspruch 14, in der $R_6$ ein 3-(2-Aminoäthyl)-2-oxo-1-imidazolidinylgruppe ist.

16. Verfahren zur Herstellung einer Verbindung nach Anspruch 6, in der $R_1$ ein (Z)-2-Amino-α-($C_1$—$C_{10}$-alkoxyimino)-4-thiazolacetylrest ist.

17. Verfahren zur Herstellung einer Verbindung nach Anspruch 16, in der $R_1$ ein (Z)-2-Amino-α-(methoxyimino)-4-thiazolacetylgruppe ist.

18. Verfahren zur Herstellung einer Verbindung nach Anspruch 16, in der $R_1$ ein (Z)-2-Amino-α-[(1-carboxy-1-methyläthoxy)imino]-4-thiazolacetylgruppe ist.

19. Verfahren nach Anspruch 1 und 2 zur Herstellung von [3S(Z)]-2-[[[1-(2-Amino-4-thiazolyl)-2-[[1-[[[[(3-äthyl-2-oxo-1-imidazolidinyl)carbonyl]-amino]sulfonyl]amino]carbonyl]-2-oxo-3-azetidinyl])-amino]-2-oxoäthyliden]amino]oxy]-2-methylpropionsäure oder einem Salz davon.

20. Verfahren nach Anspruch 1 und 2 zur Herstellung von [3S(Z)]-2-[[[1-(2-Amino-4-thiazolyl)-2-[[1-[[[[(3-äthyl-2-oxo-1-imidazolidinyl)carbonyl]-amino]sulfonyl]amino]carbonyl]-2-oxo-3-azetidinyl])-amino]-2-oxoäthyliden]amino]oxy]-2-methylpropionsäure-dikaliumsalz.

21. Verfahren nach Anspruch 1 und 2 zur Herstellung von [3S(Z)]-2-[[[2-[[1-[[[[[3-(2-Aminoäthyl)-2-oxo-1-imidazolidinyl]-carbonyl]-amino]sulfonyl]-amino]carbonyl)-2-oxo-3-azetidinyl]amino]-1-(2-amino-4-thiazolyl)-2-oxoäthyliden]amino]oxy]-2-methylpropionsäure oder einem Salz davon.

22. Verfahren nach Anspruch 1 und 2 zur Herstellung von [3S(Z)]-2-[[[2-[[1-[[[[[3-(2-Aminoäthyl)-2-oxo-1-imidazolidinyl]carbonyl]amino]sulfonyl]-amino]carbonyl)-2-oxo-3-azetidinyl]amino]-1-(2-amino-4-thiazolyl)-2-oxoäthyliden]amino]oxy]-2-methylpropionsäure-dikaliumsalz.

23. Verfahren nach Anspruch 1 und 2 zur Herstellung von [3S(Z)]-2-[[[2-[[1-[[[[(3-Methyl-2-oxo-1-imidazolidinyl)carbonyl]amino]sulfonyl]amino]-carbonyl]-2-oxo-3-azetidinyl]amino]-1-(2-amino-4-thiazolyl)-2-oxoäthyliden]amino]oxy]-2-methylpropionsäure oder ein Salz davon.

24. Verfahren nach Anspruch 1 und 2 zur Herstellung von [3S(Z)]-2-[[[2-[[1-[[[[(3-Methyl-2-oxo-1-imidazolidinyl)carbonyl]amino]sulfonyl]amino]-carbonyl]-2-oxo-3-azetidinyl]amino]-1-(2-amino-4-thiazolyl)-2-oxoäthyliden]amino]oxy]-2-methyl-propionsäure-dikaliumsalz.

25. Verfahren nach Anspruch 1 und 2 zur Herstellung von [3S(Z)]-2-[[[2-[[1-[[[[(2-Oxo-1-imidazolidinyl)-carbonyl]amino]sulfonyl]amino]carbonyl]-2-oxo-3-azetidinyl]amino]-1-(2-amino-4-thiazolyl)-2-oxoäthyliden]amino]oxy]-2-methylpropionsäure oder einem Salz davon.

26. Verfahren nach Anspruch 1 und 2 zur Herstellung von [3S(Z)]-2-[[[2-[[1-[[[[(2-Oxo-1-imidazolidinyl)-carbonyl]amino]sulfonyl]amino]carbonyl]-2-oxo-3-azetidinyl]amino]-1-(2-amino-4-thiazolyl)-2-oxoäthyliden]amino]oxy]-2-methylpropionsäure-dikaliumsalz.

**Revendications pour les etats Contractants: BE CH DE FR GB IT LI LU NL SE**

1. Composé de formule

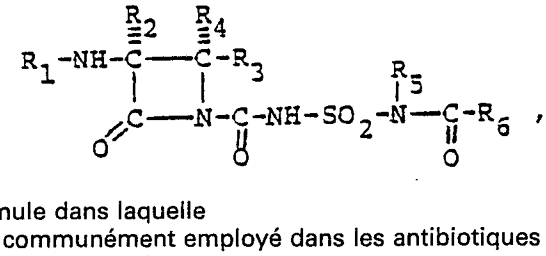 ,

ou sel de ce composé, formule dans laquelle

$R_1$ est un radical acyle communément employé dans les antibiotiques de la série des β-lactames;

$R_2$ est l'hydrogène ou un radical méthoxy;

$R_3$ et $R_4$ sont identiques ou différents et sont chacun l'hydrogène ou un radical alkyle ou aryle;

$R_5$ est l'hydrogène ou un radical alkyle ou aryle;

$R_6$ est l'hydrogène, un radical alkyle ou aryle, un hétérocycle à 5, 6 ou 7 chaînons, $-NR_7R_8$, ou $-(CH_2)_n-X$ où n est égal à 1, 2, 3 ou 4 et X est un atome d'halogène, un radical aryle, alcoxy ou aryloxy ou $-NR_9R_{10}$;

$R_7$ et $R_8$ sont identiques ou différents et sont chacun un atome d'hydrogène ou un radical alkyle ou aryle, ou bien $R_7$ est un atome d'hydrogène et $R_8$ est un hétérocycle à 5, 6 ou 7 chaînons ou $-(CH_2)_n-Y$ où n est égal à 1, 2, 3 ou 4 et Y est un groupement alcoxy, amine ou alkylthio ou un atome d'halogène; et

$R_9$ et $R_{10}$ sont identiques ou différents et sont chacun un atome d'hydrogène ou un radical alkyle, ou bien $R_9$ est un atome d'hydrogène et $R_{10}$ est un hétérocycle à 5, 6 ou 7 chaînons, et dans laquelle les définitions ci-dessus sont les suivantes:

les radicaux alkyle et alcoxy sont des radicaux de 1 à 10 atomes de carbone,

le radical aryle est un radical phényle éventuellement substitué par 1, 2 ou 3 atomes ou groupements amine ($-NH_2$), halogène, hydroxyle, trifluorométhyle, alkyle en $C_1-C_4$, alcoxy en $C_1-C_4$ ou carboxyle, et les hétérocycles à 5, 6 ou 7 chaînons sont des radicaux aromatiques ou non aromatiques contenant un ou plusieurs atomes d'azote, d'oxygène ou de soufre, et, à condition que $R_6$ soit $NR_7R_8$ ou un hétérocycle, facultativement substitués par oxo(=O), halogène, hydroxy, nitro, amine, cyano, trifluorométhyle, alkyle en $C_1-C_4$, alcoxy en $C_1-C_4$, alkylsulfonyle, phényle, phényle substitué par 1, 2 ou 3 atomes ou groupements choisis parmi les groupements amine ($-NH_2$), halogène, hydroxyle, trifluorométhyle, alkyle en $C_1-C_4$, alcoxy en $C_1-C_4$ et carboxyle, 2-furylméthylène-imine

(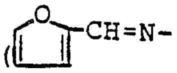),

phénylméthylène-imine et alkyle en $C_1-C_4$, ces derniers étant substitués par un ou plusieurs atomes ou groupements azide, amine ($-NH_2$), halogène, hydroxy, carboxy, cyano, alcoxycarbonyle, aminocarbonyle, alcanoyloxy de 2 à 10 atomes de carbone, alcoxy, aryloxy, hétérocycle-oxy à 5, 6 ou 7 chaînons, non substitué, mercapto, alkylthio, arylthio, alkylsulfinyle, ou alkylsulfonyle, où les fragments aryle, alcoxy et alkyle sont tels que définis ci-dessus.

2. Composé de formule

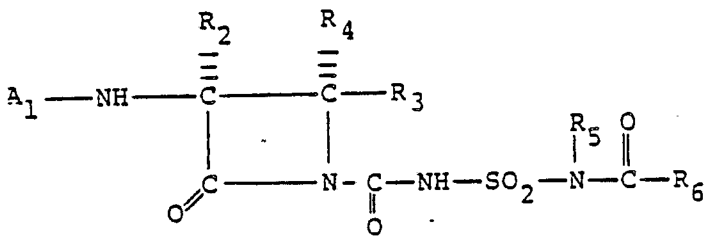

dans laquelle $R_2$, $R_3$, $R_4$, $R_5$ et $R_6$ sont tels que définis dans la revendication 1 et $A_1-NH-$ est un groupement amine protégé ou bien $A_1-$ est un atome d'hydrogène.

3. Composé selon les revendications 1 et 2, dans lequel $R_2$ est un atome d'hydrogène.

4. Composé selon les revendications 1 et 2, dans lequel $R_5$ est un atome d'hydrogène.

5. Composé selon les revendications 1 et 2, dans lequel $R_3$ et $R_4$ sont chacun un atome d'hydrogène.

6. Composé selon les revendications 1 et 2, dans lequel $R_2$, $R_3$, $R_4$ et $R_5$ sont chacun un atome d'hydrogène.

7. Composé selon la revendication 6, dans lequel $R_6$ est un atome d'hydrogène.

8. Composé selon la revendication 6, dans lequel $R_6$ est un radical alkyle en $C_1-C_{10}$.

9. Composé selon la revendication 6, dans lequel $R_6$ est un radical aryle tel qu'il est défini dans la revendication 1.

10. Composé selon la revendication 6, dans lequel $R_6$ est un hétérocycle à 5, 6 ou 7 chaînons.

11. Composé selon la revendication 6, dans lequel $R_6$ est un radical 4-alkyl en $C_1-C_{10}$-2,3-dioxo-1-pipérazinyle.

12. Composé selon la revendication 6, dans lequel $R_6$ est le radical 2-oxo-1-imidazolidinyle.

13. Composé selon la revendication 6, dans lequel $R_6$ est un radical 3-alkyl en $C_1-C_{10}$-2-oxo-1-imidazolidinyle.

14. Composé selon la revendication 6, dans lequel $R_6$ est un radical 3-(alkyl en $C_1-C_{10}$ substitué)-2-oxo-1-imidazolidinyle.

15. Composé selon la revendication 14, dans lequel $R_6$ est le radical 3-(2-aminoéthyl)-2-oxo-1-imidazolidinyle.

16. Composé selon la revendication 6, dans lequel $R_1$ est un radical (Z)-2-amino-α-(alcoxyimino en $C_1-C_{10}$)-4-thiazole acétyle.

17. Composé selon la revendication 16, dans lequel $R_1$ est le radical (Z)-2-amino-α-(méthoxyimino)-4-thiazole acétyle.

18. Composé selon la revendication 16, dans lequel $R_1$ est le radical (Z)-2-amino-α-[(1-carboxy-1-méthyléthoxy)imino]-4-thiazole acétyle.

19. Acide [3S(Z)]-2-[[1-(2-amino-4-thiazolyl)-2-[[1-[[[[(3-éthyl-2-oxo-1-imidazolidinyl)carbonyl]amino]-

sulfonyl]amino]carbonyl]-2-oxo-3-azétidinyl]amino]-2-oxoéthylidène]amino]oxy]-2-méthylpropanoïque, ou sel de cet acide.

20. Sel dipotassique de l'acide [3S(Z)]-2-[[[1-(2-amino-4-thiazolyl)-2-[[1-[[[[(3-éthyl-2-oxo-1-imidazolidinyl)carbonyl]amino]sulfonyl]amino]carbonyl]-2-oxo-3-azétidinyl]amino]-2-oxoéthylidène]-amino]oxy]-2-méthylpropanoïque.

21. Acide [3S(Z)]-2-[[[2-[[1-[[[[[3-(2-aminoéthyl)-2-oxo-1-imidazolidinyl)carbonyl]amino]sulfonyl]-amino]carbonyl]-2-oxo-3-azétidinyl]amino]-1-(2-amino-4-thiazolyl)-2-oxoéthylidène]amino]oxy]-2-méthylpropanoïque, ou sel de cet acide.

22. Sel dipotassique de l'acide [3S(Z)]-2-[[[2-[[1-[[[[[3-(2-aminoéthyl)-2-oxo-1-imidazolidinyl)carbonyl]-amino]sulfonyl]amino]carbonyl]-2-oxo-3-azétidinyl]amino]-1-(2-amino-4-thiazolyl)-2-oxoéthylidène]-amino]oxy]-2-méthylpropanoïque.

23. Acide [3S(Z)]-2-[[[2-[[1-[[[[(3-méthyl-2-oxo-1-imidazolidinyl)carbony]amino]sulfonyl]amino]-carbonyl]-2-oxo-3-azétidinyl]amino]-1-(2-amino-4-thiazolyl)-2-oxoéthylidène]amino]oxy]-2-méthylpropanoïque, ou sel de cet acide.

24. Sel dipotassique de l'acide [3S(Z)]-2-[[[2-[[1-[[[[(3-méthyl-2-oxo-1-imidazolidinyl)carbonyl]amino]-sulfonyl]amino]carbonyl]-2-oxo-3-azétidinyl]amino]-1-(2-amino-4-thiazolyl)-2-oxoéthylidène]amino]oxy]-2-méthylpropanoïque.

25. Acide [3S(Z)]-2-[[[2-[[1-[[[[2-oxo-1-imidazolidinyl)carbonyl]amino]sulfonyl]amino]carbonyl]-2-oxo-3-azétidinyl]amino]-1-(2-amino-4-thiazolyl)-2-oxoéthylidène]amino]oxy]-2-méthylpropanoïque, ou sel de cet acide.

26. Sel dipotassique de l'acide [3S(Z)]-2-[[[2-[[1-[[[[2-oxo-1-imidazolidinyl)carbonyl]amino]sulfonyl]-amino]carbonyl]-2-oxo-3-azétidinyl]amino]-1-(2-amino-4-thiazolyl)-2-oxoéthylidène]amino]oxy]-2-méthylpropanoïque.

27. Composé selon les revendications 1 à 26, destiné au traitement des infections bactériennes.

28. Compositions pharmaceutiques comprenant au moins un composé selon la revendication 1 ainsi que les porteurs, les additifs et les adjuvants habituels.

29. Procédé de préparation d'un composé de formule

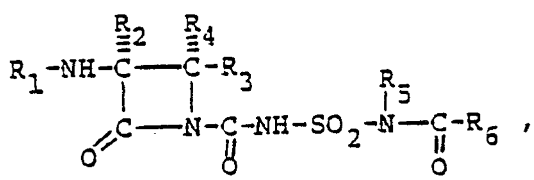

ou d'un sel de celui-ci, formule dans laquelle $R_1$, $R_2$, $R_3$, $R_4$, $R_5$ et $R_6$ sont tels que définis dans la revendication 1, qui consiste à faire réagir un β-lactame de formule

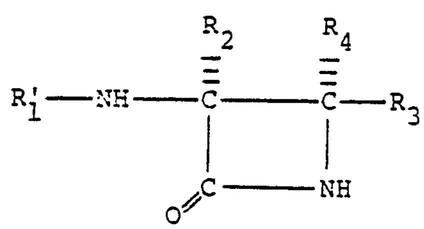

dans laquelle $R_2$, $R_3$ et $R_4$ sont tels que définis précédemment et $R_1'$ est un radical acyle ($R_1$) tel que défini précédemment ou bien le groupement $R_1'$—NH est un groupement amine protégé, avec un isocyanate de formule

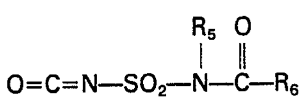

ou avec des segments séquentiels de celui-ci, formule dans laquelle $R_5$ et $R_6$ sont tels que définis précédemment, et dans le cas où $R_1'$—NH— est un groupement amine protégé, à éliminer ledit groupement protecteur et à acyler à l'aide du groupement d'acylation approprié pour former le produit voulu.

30. Procédé de préparation d'un composé de formule

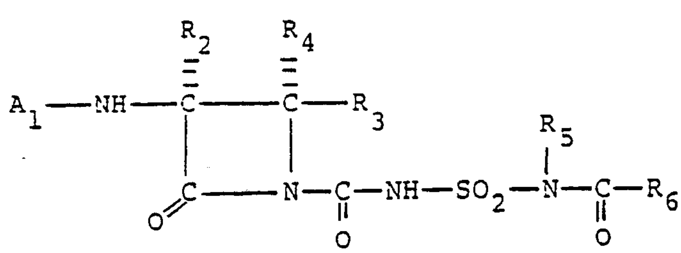

dans laquelle $R_2$, $R_3$, $R_4$, $R_5$ et $R_6$ sont tels que définis dans la revendication 1 et $A_1$—NH— est un groupement amine protégé ou bien $A_1$ est un atome d'hydrogène, caractérisé en ce qu'on fait réagir un composé de formule

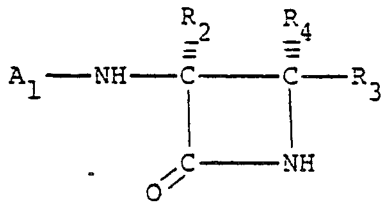

dans laquelle le groupement $A_1$—NH— est un groupement amine protégé, avec un isocyanate de formule

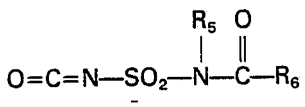

ou avec des segments séquentiels de celui-ci,

pour former un produit dans lequel $A_1$—NH— est un groupement amine protégé, et en ce qu'on élimine ledit groupement protecteur pour former un produit dans lequel $A_1$— est un atome d'hydrogène.

**Revendications pour l'etat Contractant: AT**

1. Procédé de préparation d'un composé de formule

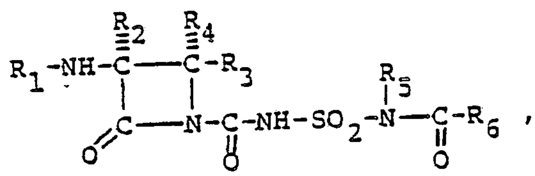

ou d'un sel de celui-ci, formule dans laquelle

$R_1$ est un radical acyle communément employé dans les antibiotiques de la série des β-lactames;

$R_2$ est l'hydrogène ou un radical méthoxy;

$R_3$ et $R_4$ sont identiques ou différents et sont chacun l'hydrogène ou un radical alkyle ou aryle;

$R_5$ est l'hydrogène ou un radical alkyle ou aryle;

$R_6$ est l'hydrogène, un radical alkyle ou aryle, un hétérocycle à 5, 6 ou 7 chaînons, $—NR_7R_8$, ou $—(CH_2)_n—X$ où n est égal à 1, 2, 3 ou 4 et X est un atome d'halogène, un radical aryle, alcoxy ou aryloxy ou $—NR_9R_{10}$;

$R_7$ et $R_8$ sont identiques ou différents et sont chacun un atome d'hydrogène ou un radical alkyle ou aryle, ou bien $R_7$ est un atome d'hydrogène et $R_8$ est un hétérocycle à 5, 6 ou 7 chaînons ou $—(CH_2)_n—Y$ où n est égal à 1, 2, 3 ou 4 et Y est un groupement alcoxy, amine ou alkylthio ou un atome d'halogène; et

$R_9$ et $R_{10}$ sont identiques ou différents et sont chacun un atome d'hydrogène ou un radical alkyle, ou bien $R_9$ est un atome d'hydrogène et $R_{10}$ est un hétérocycle à 5, 6 ou 7 chaînons, et dans laquelle les définitions ci-dessus sont les suivantes:

les radicaux alkyle et alcoxy sont des radicaux de 1 à 10 atomes de carbone,

le radical aryle est un radical phényle éventuellement substitué par 1, 2 ou 3 atomes ou groupements amine ($—NH_2$), halogène, hydroxyle, trifluorométhyle, alkyle en $C_1—C_4$, alcoxy en $C_1—C_4$ ou carboxyle, et les hétérocycles à 5, 6 ou 7 chaînons sont des radicaux aromatiques ou non aromatiques contenant un ou plusieurs atomes d'azote, d'oxygène ou de soufre, et, à condition que $R_6$ soit $NR_7R_8$ ou un hétérocycle, facultativement substitués par oxo(=O), halogène, hydroxy, nitro, amine, cyano, trifluorométhyle, alkyle en $C_1—C_4$, alcoxy en $C_1—C_4$, alkylsulfonyle, phényle, phényle substitué par 1, 2 ou 3 atomes ou groupements choisis parmi les groupements amine ($—NH_2$), halogène, hydroxyle, trifluorométhyle, alkyle en $C_1—C_4$, alcoxy en $C_1—C_4$ et carboxyle, 2-furylméthylène-imine

( 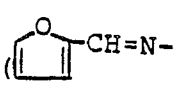 ),

phénylméthylène-imine et alkyle en $C_1—C_4$, ces derniers étant substitués par un ou plusieurs atomes ou groupements azide, amine ($—NH_2$), halogène, hydroxy, carboxy, cyano, alcoxycarbonyle, aminocarbonyle, alcanoyloxy de 2 à 10 atomes de carbone, alcoxy, aryloxy, hétérocycle-oxy à 5, 6 ou 7 chaînons, non substitué, mercapto, alkylthio, arylthio, alkylsulfinyle, ou alkylsulfonyle, où les fragments aryle, alcoxy et alkyle sont tels que définis ci-dessus;

qui consiste à faire réagir un β-lactame de formule

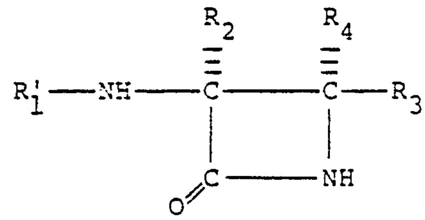

dans laquelle $R_2$, $R_3$ et $R_4$ sont tels que définis précédemment et $R_1'$ est un radical acyle ($R_1$) tel que défini précédemment ou bien le groupement $R_1'$—NH est un groupement amine protégé, avec un isocyanate de formule

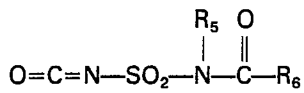

ou avec des segments séquentiels de celui-ci, formule dans laquelle $R_5$ et $R_6$ sont tels que définis précédemment, et dans le cas où $R_1'$—NH— est un groupement amine protégé, à éliminer ledit groupement protecteur et à acyler à l'aide du groupement d'acylation approprié pour former le produit voulu.

2. Procédé de préparation d'un composé de formule

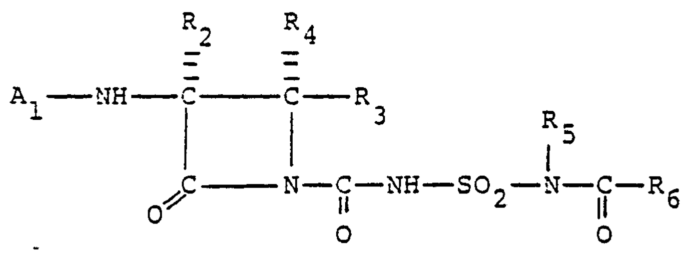

dans laquelle $R_2$, $R_3$, $R_4$, $R_5$ et $R_6$ sont tels que définis dans la revendication 1 et $A_1$—NH— est un groupement amine protégé ou bien $A_1$— est un atome d'hydrogène, caractérisé en ce qu'on fait réagir un composé de formule

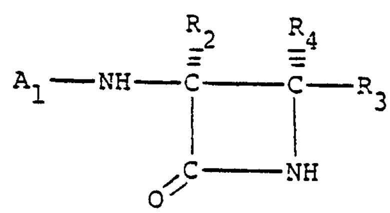

dans laquelle le groupement $A_1$—NH— est un groupement amine protégé, avec un isocyanate de formule

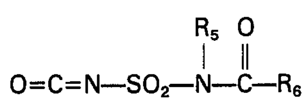

ou avec des segments séquentiels de celui-ci,

pour former un produit dans lequel $A_1$—NH— est un groupement amine protégé, et on élimine ledit groupement protecteur pour former un produit dans lequel $A_1$— est l'hydrogène.

3. Procédé de préparation d'un composé selon les revendications 1 et 2, dans lequel $R_2$ est un atome d'hydrogène.

4. Procédé de préparation d'un composé selon les revendications 1 et 2, dans lequel $R_5$ est un atome d'hydrogène.

5. Procédé de préparation d'un composé selon les revendications 1 et 2, dans lequel $R_3$ et $R_4$ sont chacun un atome d'hydrogène.

6. Procédé de préparation d'un composé selon les revendications 1 et 2, dans lequel $R_2$, $R_3$, $R_4$ et $R_5$ sont chacun un atome d'hydrogène.

7. Procédé de préparation d'un composé selon la revendication 6, dans lequel $R_6$ est un atome d'hydrogène.

8. Procédé de préparation d'un composé selon la revendication 6, dans lequel $R_6$ est un radical alkyle en $C_1$—$C_{10}$.

9. Procédé de préparation d'un composé selon la revendication 6, dans lequel $R_6$ est un radical aryle tel qu'il est défini dans la revendication 1.

10. Procédé de préparation d'un composé selon la revendication 6, dans lequel $R_6$ est un hétérocycle à 5, 6 ou 7 chaînons.

11. Procédé de préparation d'un composé selon la revendication 6, dans lequel $R_6$ est un radical 4-alkyl en $C_1$—$C_{10}$-2,3-dioxo-1-pipérazinyle.

12. Procédé de préparation d'un composé selon la revendication 6, dans lequel $R_6$ est le radical 2-oxo-1-imidazolidinyle.

13. Procédé de préparation d'un composé selon la revendication 6, dans lequel $R_6$ est un radical 3-alkyl en $C_1$—$C_{10}$-2-oxo-1-imidazolidinyle.

14. Procédé de préparation d'un composé selon la revendication 6, dans lequel $R_6$ est un radical 3-(alkyl en $C_1$—$C_{10}$ substitué)-2-oxo-1-imidazolidinyle.

15. Procédé de préparation d'un composé selon la revendication 14, dans lequel $R_6$ est le radical 3-(2-aminoéthyl)-2-oxo-1-imidazolidinyle.

16. Procédé de préparation d'un composé selon la revendication 6, dans lequel $R_1$ est un radical (Z)-2-amino-α-(alcoxyimino en $C_1$—$C_{10}$)-4-thiazoleacétyle.

17. Procédé de préparation d'un composé selon la revendication 16, dans lequel $R_1$ est le radical (Z)-2-amino-α-(méthoxyimino)-4-thiazoleacétyle.

18. Procédé de préparation d'un composé selon la revendication 16, dans lequel $R_1$ est le radical (Z)-2-amino-α-[(1-carboxy-1-méthyléthoxy)imino]-4-thiazole acétyle.

19. Procédé selon les revendications 1 et 2, de préparation de l'acide [3S(Z)]-2-[[[1-(2-amino-4-thiazolyl)-2-[[1-[[[[(3-éthyl-2-oxo-1-imidazolidinyl)carbonyl]amino]sulfonyl]amino]carbonyl]-2-oxo-3-azétidinyl]-amino]-2-oxoéthylidène]amino]oxy]-2-méthylpropanoïque, ou sel de cet acide.

20. Procédé selon les revendications 1 et 2, de préparation du sel dipotassique de l'acide [3S(Z)]-2-[[[1-(2-amino-4-thiazolyl)-2-[[1-[[[[(3-éthyl-2-oxo-1-imidazolidinyl)carbonyl]amino]sulfonyl]amino]carbonyl]-2-oxo-3-azétidinyl]amino]-2-oxoéthylidène]amino]oxy]-2-méthylpropanoïque.

21. Procédé selon les revendications 1 et 2, de préparation de l'acide [3S(Z)]-2-[[[2-[[1-[[[[[3-(2-aminoéthyl)-2-oxo-1-imidazolidinyl)carbonyl]amino]sulfonyl]amino]carbonyl]-2-oxo-3-azétidinyl]amino]-1-(2-amino-4-thiazolyl)-2-oxoéthylidène]amino]oxy]-2-méthylpropanoïque, ou d'un sel de cet acide.

22. Procédé selon les revendications 1 et 2, de préparation du sel dipotassique de l'acide [3S(Z)]-2-[[[2-[[1-[[[[[3-(2-aminoéthyl)-2-oxo-1-imidazolidinyl)carbonyl]amino]sulfonyl]amino]carbonyl]-2-oxo-3-azétidinyl]amino]-1-(2-amino-4-thiazolyl)-2-oxoéthylidène]amino]oxy]-2-méthylpropanoïque.

23. Procédé selon les revendications 1 et 2, de préparation de l'acide [3S(Z)]-2-[[[2-[[1-[[[[(3-méthyl-2-oxo-1-imidazolidinyl)carbonyl]amino]sulfonyl]amino]carbonyl]-2-oxo-azétidinyl]amino]-1-(2-amino-4-thiazolyl)-2-oxoéthylidène]amino]oxy]-2-méthylpropanoïque, ou d'un sel de cet acide.

24. Procédé selon les revendications 1 et 2, de préparation du sel dipotassique de l'acide [3S(Z)]-2-[[[2-[[1-[[[[(3-méthyl-2-oxo-1-imidazolidinyl)carbony]amino]sulfonyl]amino]carbonyl]-2-oxo-azétidinyl]-amino]-1-(2-amino-4-thiazolyl)-2-oxoéthylidène]amino]oxy]-2-méthylpropanoïque.

25. Procédé selon les revendications 1 et 2, de préparation de l'acide [3S(Z)]-2-[[[2-[[1-[[[[(2-oxo-1-imidazolidinyl)carbonyl]amino]sulfonyl]amino]carbonyl]-2-oxo-3-azétidinyl]amino]-1-(2-amino-4-thiazolyl)-2-oxoéthylidène]amino]oxy]-2-méthylpropanoïque, ou sel de cet acide.

26. Procédé selon les revendications 1 et 2, de préparation du sel dipotassique de l'acide [3S(Z)]-2-[[[2-[[1-[[[[(2-oxo-1-imidazolidinyl)carbonyl]amino]sulfonyl]amino]carbonyl]-2-oxo-3-azétidinyl]amino]-1-(2-amino-4-thiazolyl)-2-oxoéthylidène]amino]oxy]-2-méthylpropanoïque.